# EUROPÄISCHE PATENTSCHRIFT

(11) **EP 0 513 262 B1**
(45) Veröffentlichungstag und Bekanntmachung des Hinweises auf die Patenterteilung: **05.04.2000**
(21) Anmeldenummer: 91919712.9
(22) Anmeldetag: 14.11.1991
(51) Int. Cl.: C07D 213/65, C07D 213/61, C09K 19/34

(54) **3,6-DISUBSTITUIERTE-2-FLUOROPYRIDINE**
3,6-DISUBSTITUTED 2-FLUOROPYRIDINES
2-FLUOROPYRIDINES 3,6-DISUBSTITUEES

(30) Priorität: 27.11.1990 DE 4037630
(43) Veröffentlichungstag der Anmeldung: 19.11.1992
(73) Patentinhaber: MERCK PATENT GmbH, 64271 Darmstadt (DE)
(72) Erfinder: REIFFENRATH, Volker, D-6101 Rossdorf (DE); GEELHAAR, Thomas, D-6500 Mainz (DE)
(86) Internationale Anmeldenummer: EP9102148
(87) Internationale Veröffentlichungsnummer: WO9209576

(56) Entgegenhaltungen:
- EP-A- 0 194 153
- EP-A- 0 267 670
- EP-A- 0 283 326
- EP-A- 0 385 692
- WO-A-91/04248
- WO-A-91/04249

## Beschreibung

Die Erfindung betrifft 3,6-Disubstituierte-2-Fluoropyridine der Formel I, 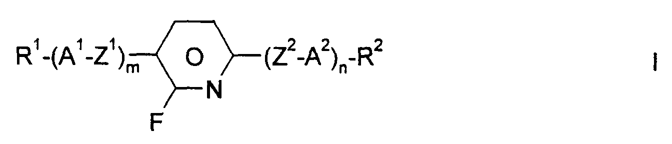 wobei
- R¹ und R²: jeweils unabhängig voneinander einen unsubstituierten oder einen einfach durch CN oder Halogen substituierten Alkyl- oder Alkenylrest mit bis zu 15 C-Atomen, wobei in diesen Resten auch eine oder mehrere CH₂-Gruppen jeweils unabhängig voneinander durch -S-, -O-, -CO-, - CO-O-, -O-CO- oder -O-CO-O-so ersetzt sein können, daß S- und/oder O-Atome nicht direkt miteinander verknüpft sind, einer der Reste R¹ und R² auch Halogen, - CF₃, -OCF₃ oder OCHF₂,
- A¹ und A²: jeweils unabhängig voneinander einen
(a) trans-1,4-Cyclohexylenrest, worin auch eine oder mehrere nicht benachbarte CH₂-Gruppen durch -O- und/oder -S- ersetzt sein können,
(b) 1,4-Phenylenrest, worin auch eine oder zwei CH-Gruppen durch N ersetzt sein können,
   wobei der Rest (a) durch CN oder Halogen und der Rest (b) durch Halogen substituiert sein können,
- Z¹ und Z²: jeweils unabhängig voneinander -CH₂-CH₂-, -C≡C-, -CH₂O-, -OCH₂-, -CO-O-, -O-CO-, -CH=N-, -CH₂S-, -SCH₂, eine Einfachbindung oder eine Alkylengruppe mit 3 bis 6 C-Atomen, worin auch eine CH₂-Gruppe durch -O-, -CO-O-, -O-CO-, -CH-Halogen- oder -CHCN- ersetzt sein kann,
- m: 0, 1 oder 2,
- n: 0, 1 oder 2, und
- m + n: 1, 2 oder 3
bedeuten,
mit den Maßgaben, daß
a) im Falle R² Halogen, -CF₃, -OCF₃, OCHF₂ oder Alkoxy,
   n 1 oder 2 ist,
b) im Falle n = 1 oder 2,
   Z² -CH₂CH₂-, -C≡C-, -CH₂O-, -CO-O- oder eine Einfachbindung bedeutet, und
c) Verbindungen ausgeschlossen sind, in denen R² Alkenyloxy, Oxaalkyloxy oder Alkylcarbonyloxy ist und n 0 bedeutet.

Chirale getiltete smektische flüssigkristalline Phasen mit ferroelektrischen Eigenschaften können hergestellt werden, indem man Basismischungen mit einer oder mehreren getilteten smektischen Phasen mit einem geeigneten chiralen Dotierstoff versetzt (L.A. Beresnev et al., Mol. Cryst. Liq. Cryst. 89, 327 (1982); H.R. Brand et al., J. Physique 44 (lett.), L 771 (1983). Solche Phasen können als Dielektrika für schnell schaltende Displays verwendet werden, die auf dem von Clark und Lagerwall beschriebenen Prinzip der SSFLC-Technologie (N.A. Clark und S.T. Lagerwall, Appl. Phys. Lett. 36, 899 (1980); USP 4,367,924) auf der Basis der ferroelektrischen Eigenschaften der chiral getilteten Phase beruhen. In dieser Phase sind die langgestreckten Moleküle in Schichten angeordnet, wobei die Moleküle einen Tiltwinkel zur Schichtennormalen aufweisen. Beim Fortschreiten von Schicht zu Schicht ändert sich die Tiltrichtung um einen kleinen Winkel bezüglich einer senkrecht zu den Schichten stehenden Achse, so daß eine Helixstruktur ausgebildet wird. In Displays, die auf dem Prinzip der SSFLC-Technologie beruhen, sind die smektischen Schichten senkrecht zu den Platten der Zelle angeordnet. Die helixartige Anordnung der Tiltrichtungen der Moleküle wird durch einen sehr geringen Abstand der Platten (ca. 1-2 µm) unterdrückt. Dadurch werden die Längsachsen der Moleküle gezwungen, sich in einer Ebene parallel zu den Platten der Zelle anzuordnen, wodurch zwei ausgezeichnete Tiltorientierungen entstehen. Durch Anlegen eines geeigneten elektrischen Wechselfeldes kann in der eine spontane Polarisation aufweisenden flüssigkristallinen Phase zwischen diesen beiden Zuständen hin- und hergeschaltet werden. Dieser Schaltvorgang ist wesentlich schneller als bei herkömmlichen verdrillten Zellen (TN-LCD's), die auf nematischen Flüssigkristallen basieren.

Ein großer Nachteil für viele Anwendungen der derzeit verfügbaren Materialien mit chiral getilteten smektischen Phasen (wie z.B. Sc*, jedoch auch S_{H}*, S_{I}*, S_{J}*, S_{K}*, S_{G}*, S_{F}*) ist deren geringe chemische, thermische und Photo-Stabilität. Eine weitere nachteilige Eigenschaft von Displays basierend auf derzeit verfügbaren chiral getilteten smektischen Mischungen ist, daß die Spontanpolarisation zu kleine Werte aufweist, so daß das Schaltzeitverhalten der Displays ungünstig beeinflußt wird und/oder der Pitch und/oder der Tilt und/oder die Viskosität der Phasen nicht den Anforderungen der Display-Technologie entspricht. Darüberhinaus ist meist der Temperaturbereich der ferroelektrischen Phasen zu klein und liegt überwiegend bei zu hohen Temperaturen.

Es wurde nun gefunden, daß die Verwendung von Verbindungen der Formel I als Komponenten chiral getilteter smektischer Mischungen die erwähnten Nachteile wesentlich vermindern kann. Die Verbindungen der Formel I sind somit als Komponenten chiral getilteter smektischer flüssigkristalliner Phasen vorzüglich geeignet. Insbesondere sind mit ihrer Hilfe chemisch besonders stabile chiral getiltete smektische flüssigkristalline Phasen mit günstigen ferroelektrischen Phasenbereichen, günstigen Weiten für die Viskosität, insbesondere mit breiten Sc* Phasenbereichen, hervorragender Unterkühlbarkeit bis zu Temperaturen unter 0 °C ohne daß Kristallisation auftritt und für derartige Phasen mittleren bis hohen Werten für die spontane Polarisation herstellbar. P ist die spontane Polarisation in nC/cm². Die Verbindungen der Formel I eignen sich jedoch auch für flüssigkristalline S_{A}-Phasen für den elektroklinen Effekt.

Die Verbindungen der Formel I weisen eine negative Anisotropie der Dielektrizitätskonstanten auf (Δε = -0,2 bis -5,0) bei gleichzeitiger großer Dielektrizitätskonstante senkrecht zur Längsachse des Moleküls (ε_{⊥}) besitzen daher einen breiten Anwendungsbereich. In Abhängigkeit von der Auswahl der Substituenten können diese Verbindungen als Basismaterialien dienen, aus denen flüssigkristalline smektische Phasen zum überwiegenden Teil zusammengesetzt sind; es können aber auch Verbindungen der Formel I flüssigkristallinen Basismaterialien aus anderen Verbindungsklassen zugesetzt werden, um beispielsweise die dielektrische und/oder optische Anisotropie und/oder die Viskosität und/oder die spontane Polarisation und/oder den Phasenbereich und/oder den Tiltwinkel und/oder den Pitch eines solchen Dielektrikums zu variieren.

Ähnliche Verbindungen sind z.B. aus der internationalen Patentanmeldung WO 88/07992 bekannt. Diese weisen eine 2-Chloro-3-cyanopyridin-6-ylgruppe auf, werden als Zwischenprodukte zur Herstellung von Verbindungen mit einer 4-Cyanopyridin-6-ylgruppe eingesetzt und eignen sich nicht als Komponenten chiral getilteter, smektischer flüssigkristalliner Medien.

EP 0 385 692 beschreibt optisch aktive Verbindungen, die einen Pyridinring enthalten können, der jedoch nicht Fluor-stubstituiert ist. EP 0 194 153 beschreibt lateral F oder Cl substituierte, terminal cyanosubstituierte Pyridinverbindungen.

Die gemäß den Maßgaben a) und b) ausgeschlossenen Verbindungen sind Gegenstand der nicht vorveröffentlichten internationalen Anmeldung PCT/EP 90/01536. Diese eignen sich nicht für ferroelektrische Anzeigen.

Die gemäß den Maßgaben b) und c) ausgeschlossenen Verbindungen sind Gegenstand der nicht vorveröffentlichten PCT/EP90/01535.

Die optisch aktiven Verbindungen der Formel I werden von der allgemeinen Formel z.B. den allgemeinen Formeln, z.B. der EP 0 267 670 und der EP-0 283 326-A umfaßt. Jedoch kann der Fachmann dieser Schrift weder die vorteilhaften Eigenschaften der erfindungsgemäßen Verbindungen noch Wege zu ihrer Herstellung entnehmen.

Der Fachmann konnte somit aus dem Stand der Technik weder in einfacher Art und Weise Synthesemöglichkeiten für die beanspruchten Verbindungen entnehmen noch erkennen, daß die erfindungsgemäßen Verbindungen überwiegend breite und günstig gelegene S_{c}-Phasen aufweisen sowie sich durch günstige Werte für die Rotationsviskosität auszeichnen.

Gegenstand der Erfindung sind somit die 2-Halogenpyridine der Formel I, insbesondere worin n 1 oder 2 ist und mindestens einer der Reste A¹ und A² gegebenenfalls durch Fluor substituiertes 1,4-Phenylen, 1,4-Cyclohexylen, Pyrimidin-2,5-diyl oder Pyridin-2,5-diyl bedeutet.

Die Verbindungen der Formel I umfassen die Verbindungen der Formel I2 und I4 mit 2 Ringen, 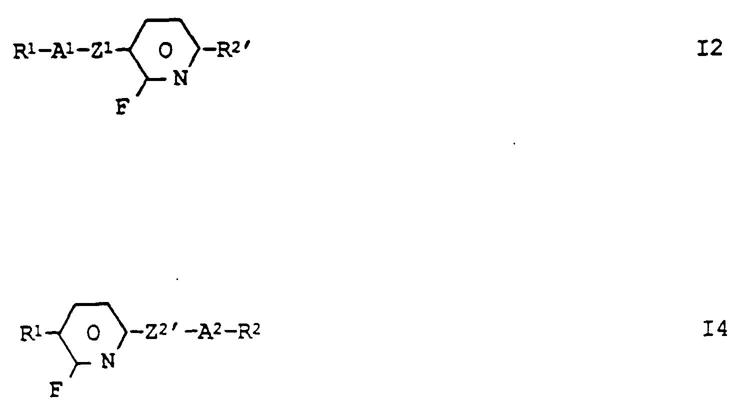 worin R¹, R², A¹, A², Z¹ und Z² die angegebene Bedeutung besitzen und
- R^{2'}: unsubstituiertes Alkyl, Oxaalkyl oder Dioxaalkyl oder einfach durch CN, Halogen oder CF₃ substituiertes Alkyl oder Alkoxy, und
- Z^{2'}: -CH₂CH₂-, -C≡C, -CH₂O-, -CO-O- oder eine Einfachbindung bedeutet,
Verbindungen der Formeln I6, I8 und I10 mit 3 Ringen, 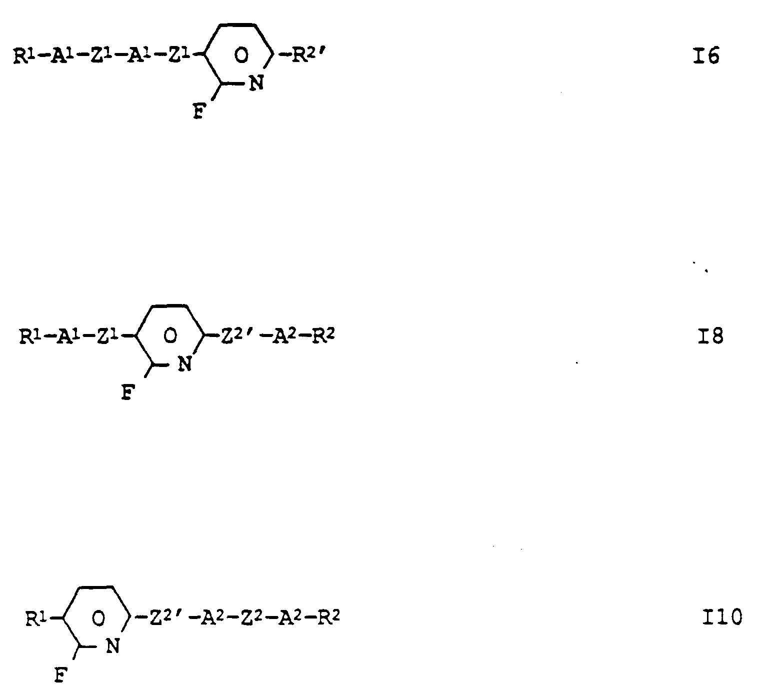 sowie Verbindungen der Formeln I11 bis I12 mit 4 Ringen.

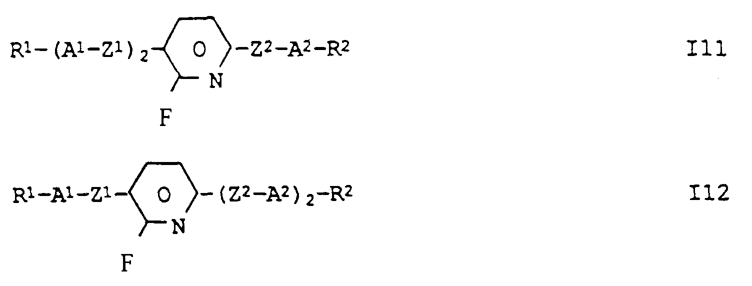

Gegenstand der Erfindung sind insbesondere solche optisch aktiven Halogenpyridine der Formel I, worin einer der Reste R¹ und R² eine chirale Gruppe der Formel III, 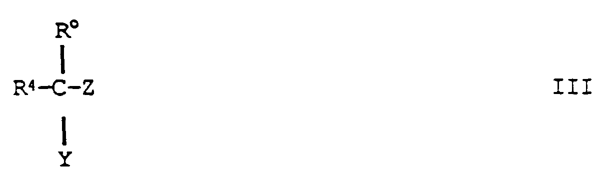 wobei
- R⁴: eine Gruppe der Formel

(CH₂)ₛ-Q³-CₒH₂ₒ₊₁,

worin
- Q³: -O-, -O-CO- oder eine Einfachbindung,
- s: 0, 1 oder 2, und
- o: 1 bis 7 ist,
- Y: CN, Halogen oder CH₃,
- Z: eine Einfachbindung oder -(CH₂)ₚ-, worin eine CH₂-Gruppe durch -O-, -O-CO- oder -CO-O- ersetzt sein kann und p 1, 2, 3, 4, 5 oder 6 ist, und
- R^{o}: H oder CH₃ bedeutet,
mit der Maßgabe, daß nur eine der Gruppen R^{o}, R⁴ und Y CH₃ bedeutet. Gegenstand der Erfindung sind ferner flüssigkristalline Phasen mit einem Gehalt an mindestens einer Verbindung der Formel I. Weiterhin sind Gegenstand der Erfindung ferroelektrische flüssigkristalline Phasen mit einem Gehalt an mindestens einer Verbindung, welche eine Gruppe der Formel 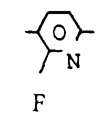 der Formel I sowie Flüssigkristallanzeigelemente, insbesondere ferroelektrische elektrooptische Anzeigeelemente, die derartige Phasen enthalten.

Die erfindungsgemäßen Phasen enthalten vorzugsweise mindestens zwei, insbesondere mindestens drei Verbindungen der Formel I. Besonders bevorzugt sind erfindungsgemäße chirale getiltete smektische flüssigkristalline Phasen, deren achirale Basismischung neben Verbindungen der Formel I mindestens eine andere Komponente mit negativer oder betragsmäßig kleiner positiver dielektrischer Anisotropie enthält. Diese weiteren Komponente(n) der achiralen Basismischung können 1 bis 50 %, vorzugsweise 10 bis 25 %, der Basismischung ausmachen. Als weitere Komponenten mit betragsmäßig kleiner positiver oder negativer dielektrischer Anisotropie eignen sich Verbindungen der Formel IV, welche die Verbindungen der Teilformeln IVa bis IVi umfaßt:

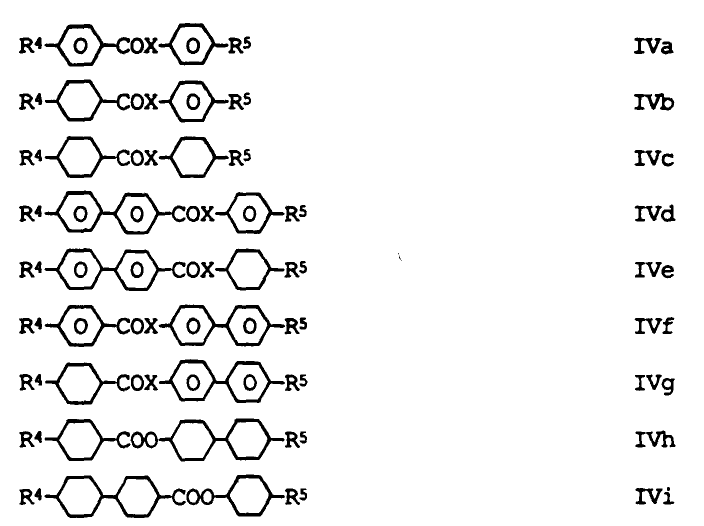

R⁴ und R⁵ sind jeweils vorzugsweise geradkettig Alkyl, Alkoxy, Alkanoyloxy oder Alkoxycarbonyl mit jeweils 3 bis 12 C-Atomen. X ist vorzugsweise O. In den Verbindungen der Formeln IVa, IVb, IVd, IVe, IVf und IVg kann auch eine 1,4-Phenylengruppe lateral durch Halogen oder CN, insbesondere bevorzugt durch Fluor, substituiert sein.

Besonders bevorzugt sind die Verbindungen der Teilformeln IVa, IVb, IVd und IVf, worin R⁴ und R⁵ jeweils geradkettiges

Alkyl oder Alkoxy mit jeweils 5 bis 10 C-Atomen bedeutet. Besonders bevorzugte Einzelverbindungen sind in der folgenden Tabelle I angegeben:

**Tabelle I**

| Formel | R⁴ | R⁵ | X |
|---|---|---|---|
| IVa | n-Decyloxy | n-Heptyloxy | 0 |
| IVa | n-Hexyloxy | n-Decyloxy | 0 |
| IVa | n-Octyloxy | n-Heptyl | 0 |
| IVa | n-Octyloxy | n-Pentyl | 0 |
| IVa | n-Decyloxy | n-Heptyl | 0 |
| IVa | n-Decyloxy | n-Pentyl | 0 |
| IVf | n-Pentyl | n-Pentyl | 0 |
| IVf | n-Pentyl | n-Hexyl | 0 |

Die Verbindungen der Teilformeln IVc, IVh und IVi eignen sich als Zusätze zur Schmelzpunktserniedrigung und werden normalerweise den Basismischungen mit nicht mehr als 5 %, vorzugsweise 1 bis 3 %, zugesetzt. R⁴ und R⁵ bedeuten in den Verbindungen der Teilformeln IVc, IVh und IVi vorzugsweise geradkettiges Alkyl mit 2 bis 7, vorzugsweise 3 bis 5, C-Atomen. Eine weitere zur Schmelzpunktserniedrigung in den erfindungsgemäßen Phasen geeignete Verbindungsklasse ist diejenige der Formel, 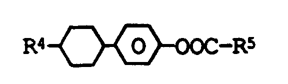 worin R⁴ und R⁵ die für IVc, IVh und IVi angegebene bevorzugte Bedeutung haben.

Als weitere Komponenten mit negativer dielektrischer Anisotropie eignen sich weiterhin Verbindungen enthaltend das Strukturelement A, B oder C.

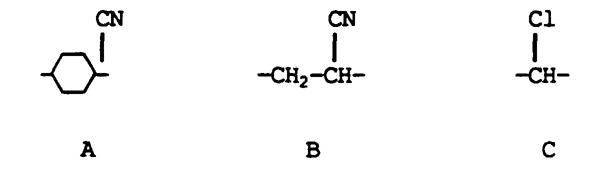

Bevorzugte Verbindungen dieser Art entsprechen den Formeln Va, Vb und Vc:

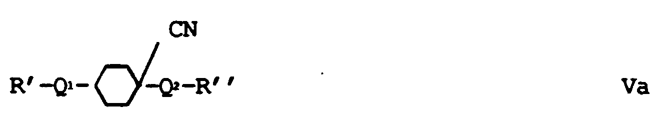

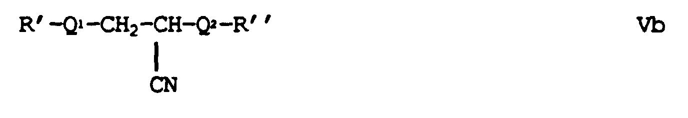

R' und R'' bedeuten jeweils vorzugsweise geradkettige Alkyl- oder Alkoxy-Gruppen mit jeweils 2 bis 10 C-Atomen. Q¹ und Q² bedeuten jeweils 1,4-Phenylen, trans-1,4-Cyclohexylen, 4,4'-Biphenylyl, 4-(trans-4-Cyclohexyl)-phenyl, trans,trans-4,4'-Bicyclohexyl oder eine der Gruppen Q¹ und Q² auch eine Einfachbindung.

Q³ und Q⁴ bedeuten jeweils 1,4-Phenylen, 4,4'-Biphenylyl oder trans-1,4-Cyclohexylen. Eine der Gruppen Q³ und Q⁴ kann auch 1,4-Phenylen bedeuten, worin mindestens eine CH-Gruppe durch N ersetzt ist. R''' ist ein optisch aktiver Rest mit einem asymmetrischen Kohlenstoffatom der Struktur 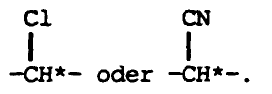 Besonders bevorzugte Verbindungen der Formel Vc sind diejenigen der Formel Vc', 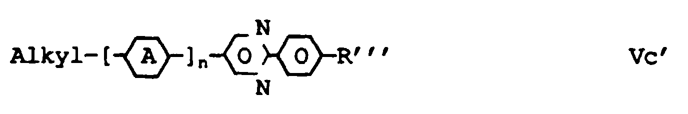 worin A 1,4-Phenylen oder trans-1,4-Cyclohexylen und n 0 oder 1 bedeutet.

Besonders bevorzugt sind solche ferroelektrischen flüssigkristalline Phasen mit einem Gehalt an mindestens einem achiralen Halogenpyridin der Formel I1, mindestens einen achiralen Phenylpyrimidin der Formel Vc', worin R''' einen Alkyl- oder Alkoxyrest mit bis zu 18 C-Atomen bedeutet, als Basismaterial mit einer breiten S_{c}-Phase und mindestens einem chiralen Halogenpyridin der Formel I als optisch aktivem Dotierstoff. Weiterhin bevorzugt sind solche ferroelektrischen flüssigkristallinen Phasen, die neben den genannten Verbindungen der Formel I1, Vc' und I mindestens ein Phenylpyridin der Formel Vd und/oder ein 2-Fluor- (L = H) bzw. 2,3-Difluorphenylpyrimidin der Formel Ve (L = F) und/oder ein Phenylpyrimidin der Formel Vf 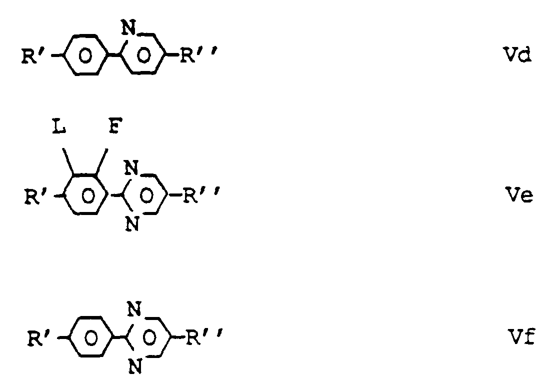 enthalten.

Die achiralen Halogenpyridine der Formel IA, 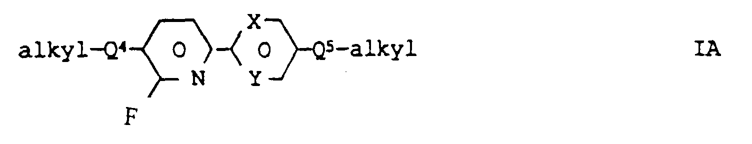 worin
- alkyl: jeweils unabhängig voneinander Alkyl mit 1 bis 18 C-Atomen,
- X und Y: jeweils unabhängig CH oder N,
- Q⁴: 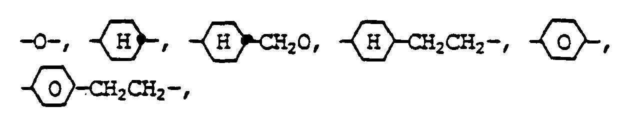 oder eine Einfachbindung und
- Q⁵: 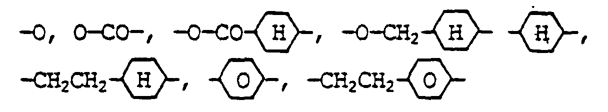 oder eine Einfachbindung bedeutet,
sind besonders als Bestandteile smektischer Basismaterialien geeignet.

Die Verbindungen der Formel IA umfassen die nachstehend angeführten achiralen bevorzugten zweikernigen und dreikernigen Materialien der Formeln IA1 bis IA15 und IA18 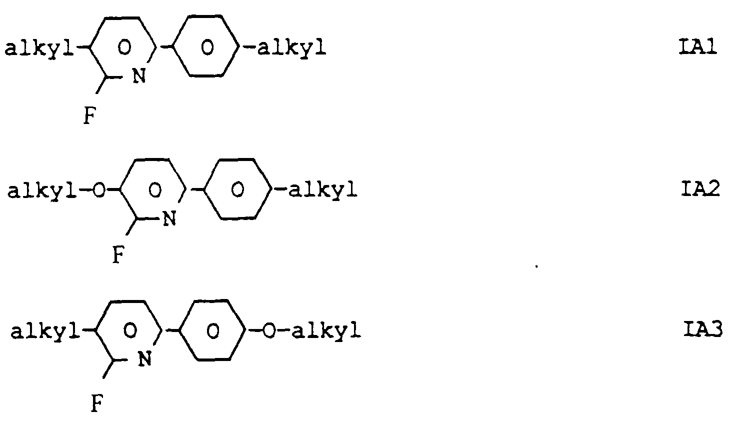 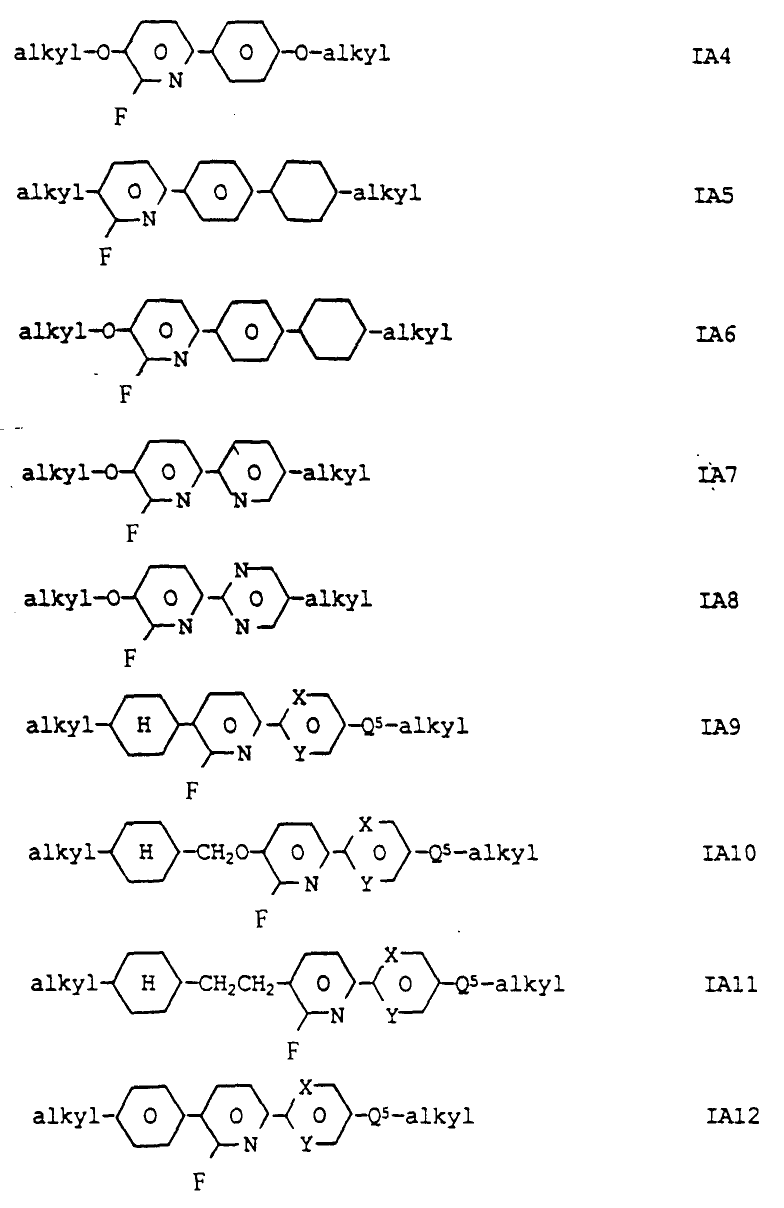 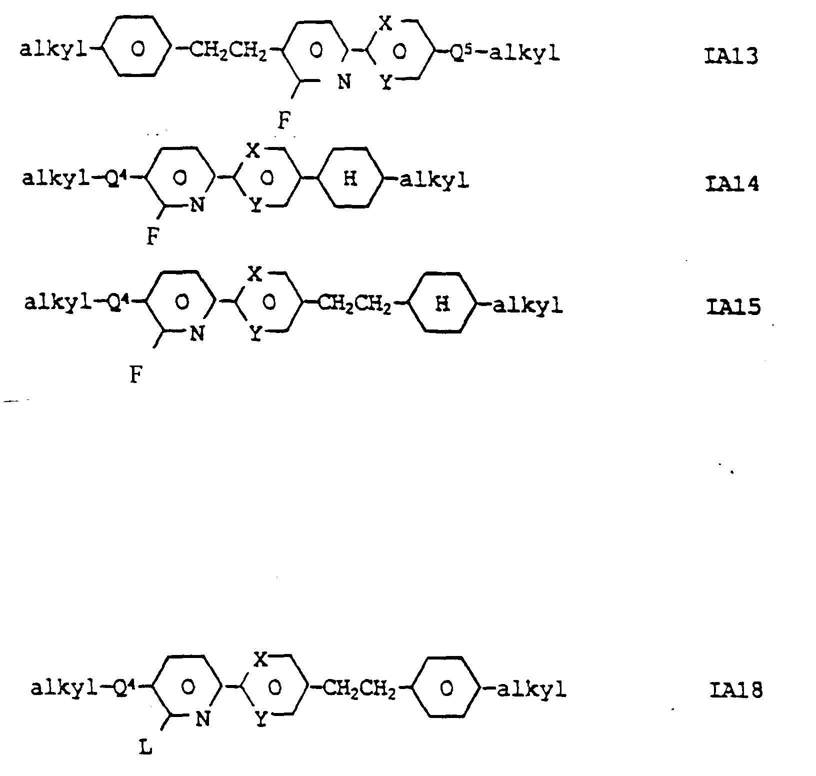 wobei bei den Verbindungen IA9 bis IA15 und IA18 Q⁴ -O- oder eine Einfachbindung und Q⁵ -O-, -O-CO- oder eine Einfachbindung bedeutet, und
- X und Y: jeweils unabhängig voneinander N oder CH, vorzugsweise CH bedeuten.

Weiterhin bevorzugte Verbindungen der Formel I sind diejenigen der Formeln IB, 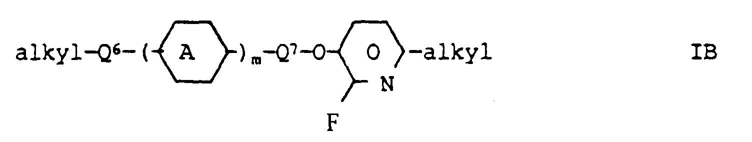 worin 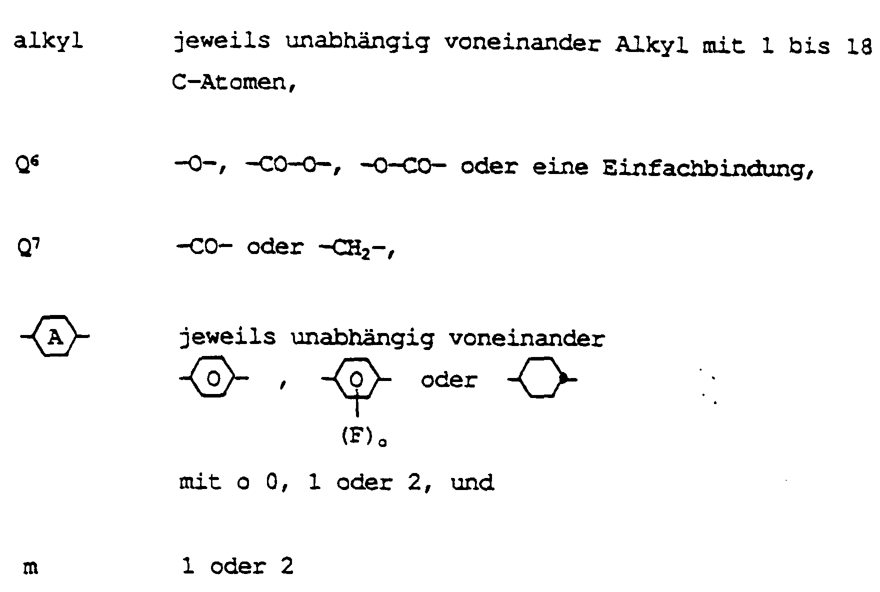 bedeuten.

Diese Verbindugnen der Formel IB sind besonderes als Bestandteile nematischer Mischungen geeignet.

Die Verbindungen der Formel IB umfassen die nachstehend aufgeführten bevorzugten zwei- und dreikernigen Materialien der Formel IB1 bis IB8:

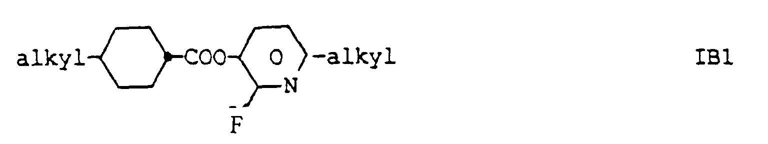

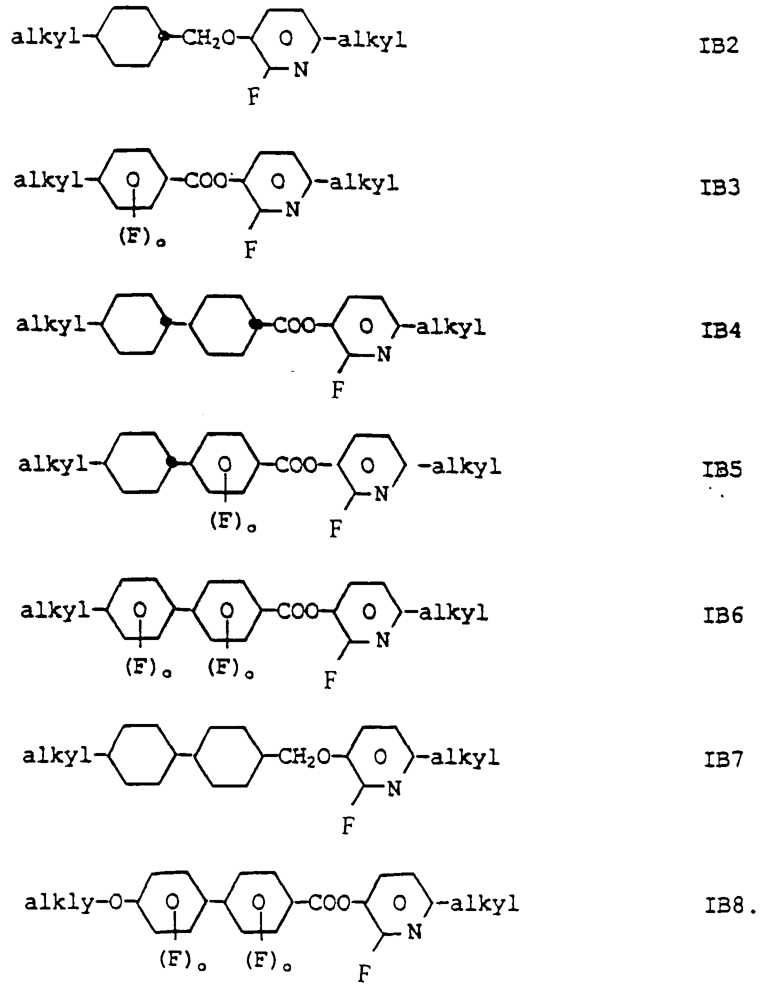

Weiterhin bevorzugte Verbindungen der Formel I sind diejenigen, worin der mit der 6-Halogenpyridin-2,5-diyl verknüpfte Rest Z¹ oder Z² eine -C≡C-Gruppe ist und A¹ und/oder A² 1,4-Phenylen, das gegebenenfalls durch 1 oder 2 Fluoratome substituiert ist, oder 1,4-Cyclohexylen bedeutet. Diese Verbindungen umfassen die bevorzugten Verbindungen der Formeln IT1 bis IT6, 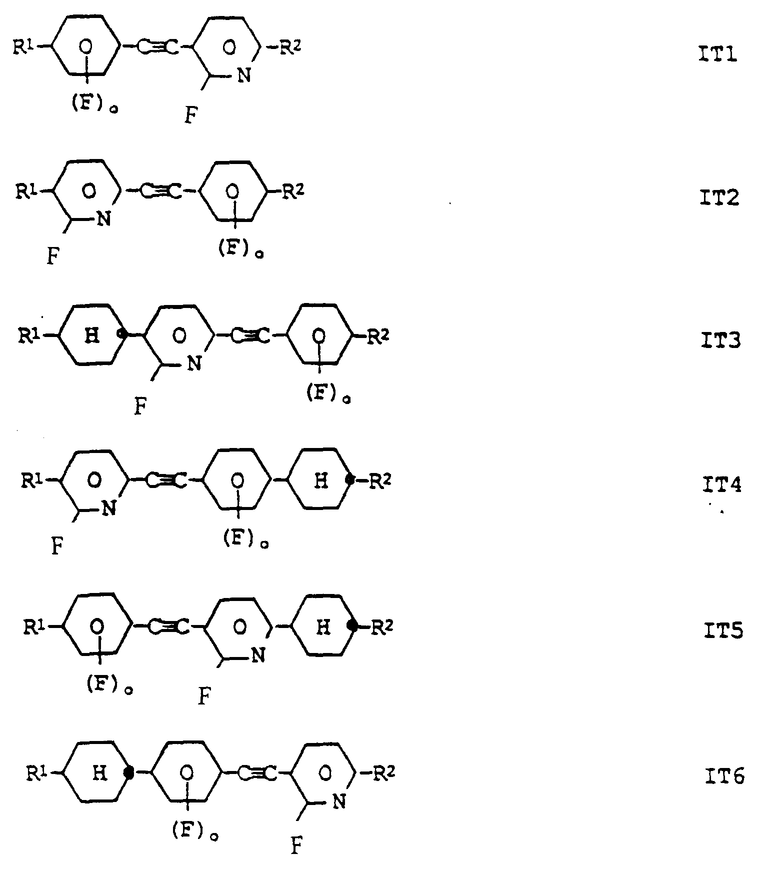 worin
- o: 0, 1 oder 2 bedeutet.

In den bevorzugten Verbindungen der Formel IT1 bis IT6 ist O vorzugsweise O; insbesondere bevorzugt sind die Verbindungen der Formel IT2 und IT4, worin R¹ Alkoxy mit 1 bis 8 C-Atomen ist.

Die Verbindung der Formel IT1 bis IT6 eignen sich insbesondere als Komponenten nematischer Phasen für ECB oder STN-Anzeigen.

R¹ und R² weisen in den vor- und nachstehenden Formeln vorzugsweise 1-13 C-Atome, insbesondere 3-12 C-Atome auf. Verbindungen der Formel I, bei denen R¹ und R² 1-7 C-Atome, vorzugsweise 1-5 C-Atome aufweisen, eignen sich besonders für flüssigkristalline Phasen für auf dem ECB-Effekt basierende Anzeigeelemente. Verbindungen der Formel I dagegen, bei denen R¹ und R² 6-15 C-Atomen, vorzugsweise 6-12 C-Atome aufweisen, eignen sich für flüssigkristalline Phasen mit ferroelektrischen Eigenschaften. In R¹ und R² können auch eine oder zwei CH₂-Gruppen ersetzt sein. Vorzugsweise ist nur eine CH₂-Gruppe ersetzt durch -O-, -S-, -CO-O- oder -O-CO-, insbesondere durch -O-.

Weiterhin bevorzugt sind solche Verbindungen, in denen 2, 3 oder nicht benachbarte CH₂-Gruppen durch -O- ersetzt sind.

In den vor- und nachstehenden Formeln bedeuten R¹ und R² vorzugsweise Alkyl, Alkoxy oder eine andere Oxaalkylgruppe.

Falls R₁ und R₂ Alkylreste, in denen auch eine ("Alkoxy" bzw. "Oxaalkyl") oder zwei ("Alkoxyalkoxy" bzw. "Dioxaalkyl") nicht benachbarte CH₂-Gruppen durch O-Atome ersetzt sein können, bedeuten, so können sie geradkettig oder verzweigt sein. Vorzugsweise sind sie geradkettig, haben 2, 3, 4, 5, 6 oder 7 C-Atome und bedeuten demnach bevorzugt Ethyl, Propyl, Butyl, Pentyl, Hexyl, heptyl, Ethoxy, Propoxy, Butoxy, Pentoxy, hexoxy oder Heptoxy, ferner Methyl, Octyl, Nonyl, Decyl, Undecyl, Dodecyl, Tridecyl, Tetradecyl, Pentadecyl, Methoxy, Octoxy, Nonoxy, Decoxy, Undecoxy, Tridecoxy oder Tetradecoxy.

Oxalkyl bedeutet vorzugsweise geradkettiges 2-Oxypropyl (= Methoxymethyl), 2- (= Ethoxymethyl) oder 3-Oxabutyl (= 2-Methoxyethyl), 2-, 3- oder 4-Oxapentyl, 2-, 3-, 4- oder 5-Oxahexyl, 2-, 3-, 4-, 5- oder 6-Oxaheptyl, 2-, 3-, 4-, 5-, 6- oder 7-Oxaoctyl, 2-, 3-, 4-, 5-, 6-, 7- oder 8-Oxanonyl, 2-, 3-, 4-, 5-, 6-, 7-, 8- oder 9-Oxadecyl, 1,3-Dioxabutyl (= Methoxymethoxy), 1,3-, 1,4-, 2,4-Dioxapentyl, 1,3-, 1,4-, 1,5-, 2,4-, 2,5- oder 3,5-Dioxahexyl, 1,3-, 1,4-, 1,5-, 1,6-, 2,4-, 2,5-, 2,6-, 3,5-, 3,6- oder 4,6-Dioxaheptyl.

Die Verbindungen der Formel I, welche eine oder zwei Dioxa- bzw. Trioxaalkylgruppe aufweisen eignen sich insbesondere als komplexbildende Podanden zur Herabsetzung der Konzentration an freien Ionen in flüssigkristallinen Mischungen, insbesondere für ferroelektrische Anzeigen. Insbesondere können mit Hilfe dieser Verbindungen sogenannte "Geisterbilder" (vgl. z.B. J. Dijon et al., SID conference, San Diego 1988, Seiten 2-249) unterdrückt werden.

Falls R¹ und R² einen Alkylrest bedeuten, in dem eine CH₂-Gruppe durch -S- ersetzt ist, so kann dieser geradkettig und verzweigt sein. Vorzugsweise ist dieser Thiaalkylrest geradkettig und bedeutet 2-Thiapropyl, 2- oder 3-Thiabutyl, 2-, 3- oder 4-Thiapentyl, 2-, 3-, 4- oder 5-Thiahexyl, 2-, 3-, 4-, 5- oder 6-Thiaheptyl, 2-, 3-, 4-, 5-, 6- oder 7-Thiaoctyl, 2-, 3-, 4-, 5-, 6-, 7- oder 8-Thianonyl oder 2-, 3-, 4-, 5-, 6-, 7-, 8- oder 9-Thiadecyl.

Besonders bevorzugt sind Alkylreste R¹ oder R², in denen die der Gruppe A¹ A² und/oder A³ benachbarte CH₂-Gruppe durch -S- ersetzt ist und bedeutet somit vorzugsweise Methylthio, Ethylthio, Propylthio, Butylthio, Pentylthio, Hexylthio, Heptylthio, Octylthio, Nonylthio oder Decylthio.

Falls R¹ und R² einen Alkenylrest bedeuten, so kan dieser geradkettig oder verzweigt sein. Vorzugsweise ist er geradkettig und hat 2 bis 10 C-Atome. Er bedeutet demnach besonders Vinyl, Prop-1-, oder Prop-2-enyl, But-1-, 2- oder But-3-enyl, Pent-1-, 2-, 3- oder Pent-4-entyl, Hex-1-, 2-, 3-, 4- oder Hex-5-enyl, Hept-1-, 2-, 3-, 4-, 5- oder Hept-6-enyl, Oct-1-, 2-, 3-, 4-, 5-, 6- oder Oct-7-enyl, Non-1-, 2-, 3-, 4-, 5-, 6-, 7- oder Non-8-enyl, Dec-1-, 2-, 3-, 4-, 5-, 6-, 7-, 8- oder Dec-9-enyl.

Falls R¹ und R² einen Alkylrest bedeuten, in dem eine CH₂- Gruppe durch -O-CO oder -CO-O- ersetzt ist, so kann dieser geradkettig oder verzweigt sein. Vorzugsweise ist er geradkettig und hat 2 bis 6 C-Atome. Er bedeutet demnach besonders Acetyloxy, Propionyloxy, Butyryloxy, Pentanoyloxy, Hexanoyloxy, Acetyloxymethyl, Propionyloxymethyl, Butyryloxymethyl, Pentaoyloxymethyl, 2-Acetyloxyethyl, 2-Propionyloxyethyl, 2-Butyryloxyethyl, 3-Acetyloxypropyl, 3-Propionyloxypropyl, 4-Acetyloxybutyl, Methoxycarbonyl, Ethoxycarbonyl, Propoxycarbonyl, Butoxycarbonyl, Pentoxycarbonyl, Methoxycarbonylmethyl, Ethoxycarbonylmethyl, Propoxycarbonylmethyl, Butoxycarbonylmethyl, 2-(Methoxycarbonyl)ethyl, 2-(Ethoxycarbonyl)ethyl, 2-(Propoxycarbonyl)ethyl, 3-(Methoxycarbonyl)propyl, 3-(Ethoxycarbonyl)propyl, 4-(Methoxycarbonyl)butyl.

Verbindungen der Formel I mit verzweigten Flügelgruppen R¹ und/oder R² können gelegentlich wegen einer besseren Löslichkeit in den üblichen flüssigkristallinen Basismaterialien von Bedeutung sein, insbesondere aber als chirale Dotierstoffe, wenn sie optisch aktiv sind. Smektische Verbindungen dieser Art eignen sich als Komponenten für ferroelektrische Materialien.

Verzweigte Gruppen dieser Art enthalten in der Regel nicht mehr als eine Kettenverzweigung. Bevorzugte verzweigte Reste R¹ und/oder R² sind Isopropyl, 2-Butyl (= 1-Methylpropyl), Isobutyl (= 2-Methylpropyl), 2-Methylbutyl, Isopentyl (= 3-Methylbutyl), 2-Methylpentyl, 3-Methylpentyl, 2-Ethylhexyl, 2-Propylpentyl, Isopropoxy, 2-Methylpropoxy, 2-Methylbutoxy, 3-Methylbutoxy, 2-Methylpentoxy, 3-Methylpentoxy, 2-Ethylhexoxy, 1-Methylhexoxy, 1-Methylheptoxy, 2-Oxa-3-methylbutyl, 3-Oxa-4-methylpentyl, 4-Methylhexyl, 2-Nonyl, 2-Decyl, 2-Dodecyl, 6-Methyloctoxy, 6-Methyloctanoyloxy, 5-Methylheptyloxycarbonyl, 2-Methylbutyryloxy, 3-Methylvaleryloxy, 4-Methylhexanoyloxy, 2-Chlorpropionyloxy, 2-Chlor-3-methylbutyryloxy, 2-Chlor-4-methylvaleryloxy, 2-Chlor-3-methylvaleryloxy, 2-Methyl-3-oxapentyl, 2-Methyl-3-oxahexyl.

Besonders bevorzugt sind die Verbindungen der Formel I mit m = 0 und R¹ und R² jeweils unabhängig voneinander Alkyl mit bis zu 15 C-Atomen, worin auch eine oder mehrere CH₂-Gruppen durch O-Atome ersetzt sein können. Vorzugsweise ist R¹ Alkoxy mit bis zu 12 C-Atomen.

Die chiralen Halogenpyridine der Formel I, worin einer der Reste R¹ und R² eine Gruppe der Formel III bedeutet, eignen sich hervorragend als Dotierstoffe zur Induktion von Ferroelektrizität in ein smektisches Basismaterial. Sie zeichnen sich insbesondere durch eine hohe Spontanpolarisation aus.

Weiterhin destabilisieren sie die smektische Phase dieser Basismaterialien nicht. Der Rest der Formel III wird im folgenden als R* bezeichnet.

Die chiralen Verbindungen der Formel I umfassen demnach die Verbindungen der Teilformeln Ic1 bis Ic4 mit 2 Ringen, 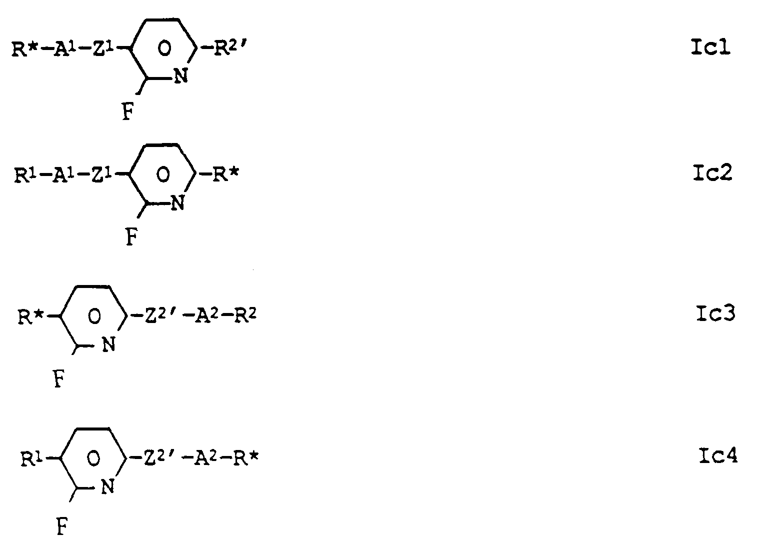 sowie die Verbindungen der Formeln Ic5 bis Ic10 mit 3 Ringen: 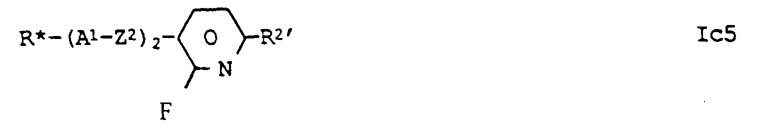 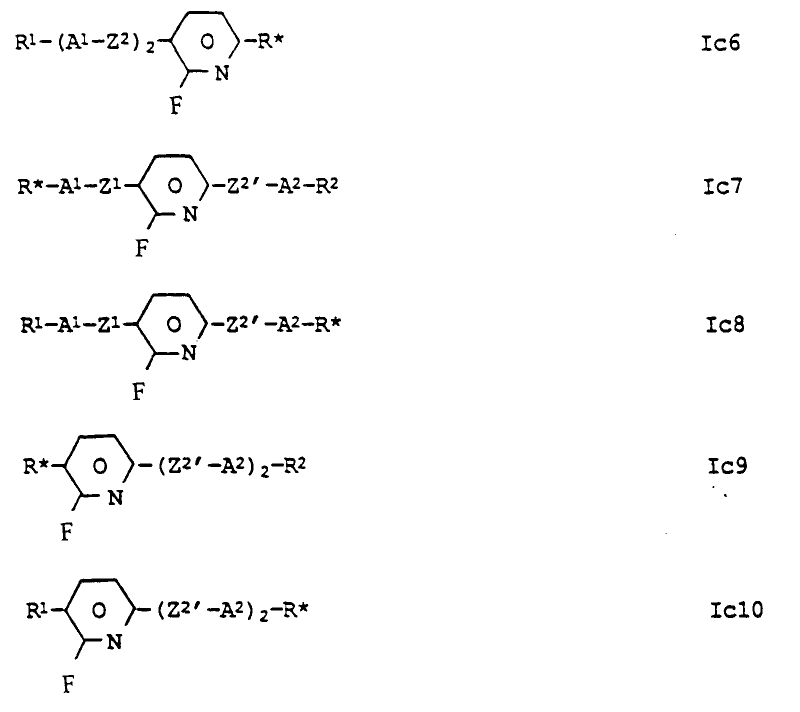 worin R¹, R², L und Z² die angegebene Bedeutung besitzen,
und R^{2'} besitzt dieselbe Bedeutung wie R², wobei jedoch Halogen, -CH₃, -OCF₃,-OCHF₂ und Alkoxy ausgeschlossen sind. Z^{2'} bedeutet -CH₂CH₂-, -C≡C-, -CH₂O-, -CO-O- oder eine Einfachbindung.

Insbesondere bevorzugt sind die der Teilformeln Ic1 bis Ic4, worin R¹, R² jeweils Alkyl oder Alkoxy mit 1 bis 12 C-Atomen, bzw. R^{2'} Alkyl, Oxaalkyl oder Dioxaalkyl mit 1 bis 12 C-Atomen bedeutet, und A¹ bzw. A² 1,4-Phenylen bedeutet, worin auch 1 oder 2 CH-Gruppen durch N ersetzt sein können.

Darunter sind die chiralen Verbindungen der Formel I besonders bevorzugt, worin R* eine Gruppe der Formel IIIa bedeutet, 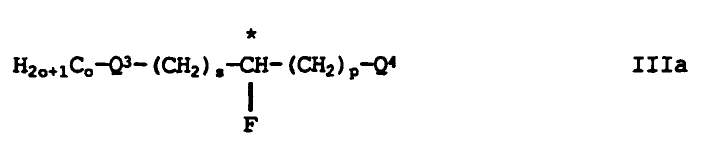 worin o und Q³ die angegebene Bedeutung besitzen, Q⁴ -O-, -CO-O- oder eine Einfachbindung bedeutet, r 1 oder 2 und p 0 oder 1 ist.

Die chiralen Reste der Formel IIIa umfassen demgemäß die chiralen Monofluoralkyl-, Monofluoraoxaalkyl- und Alkanoyloxymonofluoralkylgruppe R_{f}* der Formeln IIIa1 bis IIIa6:

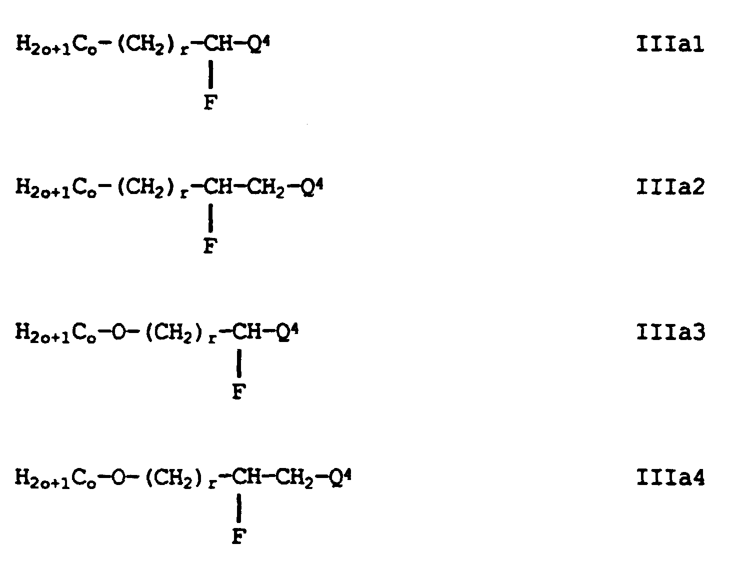

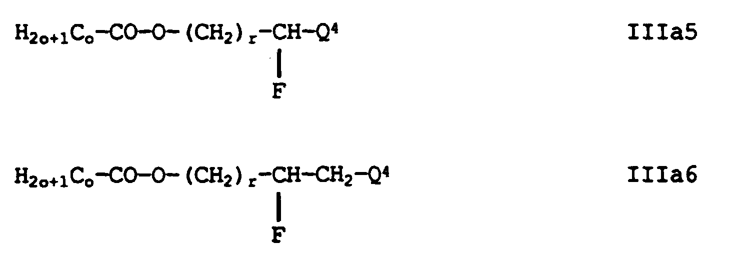

Von den chiralen Monofluorgruppen R_{f}* der Formeln IIIa1 bis IIIa6 sind diejenigen der Formeln IIIa1, IIIa3 und IIIa5 besonders bevorzugt, insbesondere diejenigen worin r 2 bedeutet.

Weiterhin bevorzugt sind die chiralen Verbindungen der Formel I, worin R* eine Gruppe der Formel IIIb 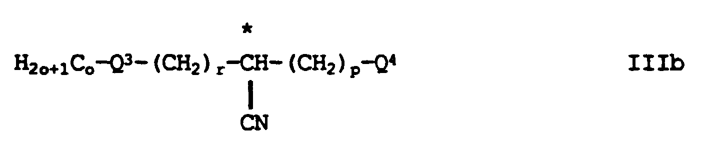 bedeutet, worin o, Q³ und Q⁴ die angegebene Bedeutung besitzen, r 1 oder 2 ist und p 0 oder 1 ist.

Weiterhin bevorzugt sind die Verbindungen der Formel I worin R* eine Gruppe der Formel IIIc 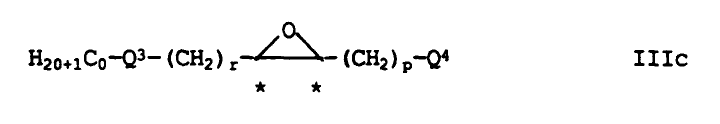 worin Q³, Q⁴, r und p die angegebene Bedeutung besitzen.

Die Verbindungen der Formel I werden nach an sich bekannten Methoden, wie sie in der Literatur (z.B. in den Standardwerken wie Houben-Weyl, Methoden der Organischen Chemie, Georg-Thieme-Verlag, Stuttgart) beschrieben sind, hergestellt.

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt.

Die erfindungsgemäßen Verbindungen lassen sich nach folgenden Reaktionsschemata (1-7) einfach herstellen.

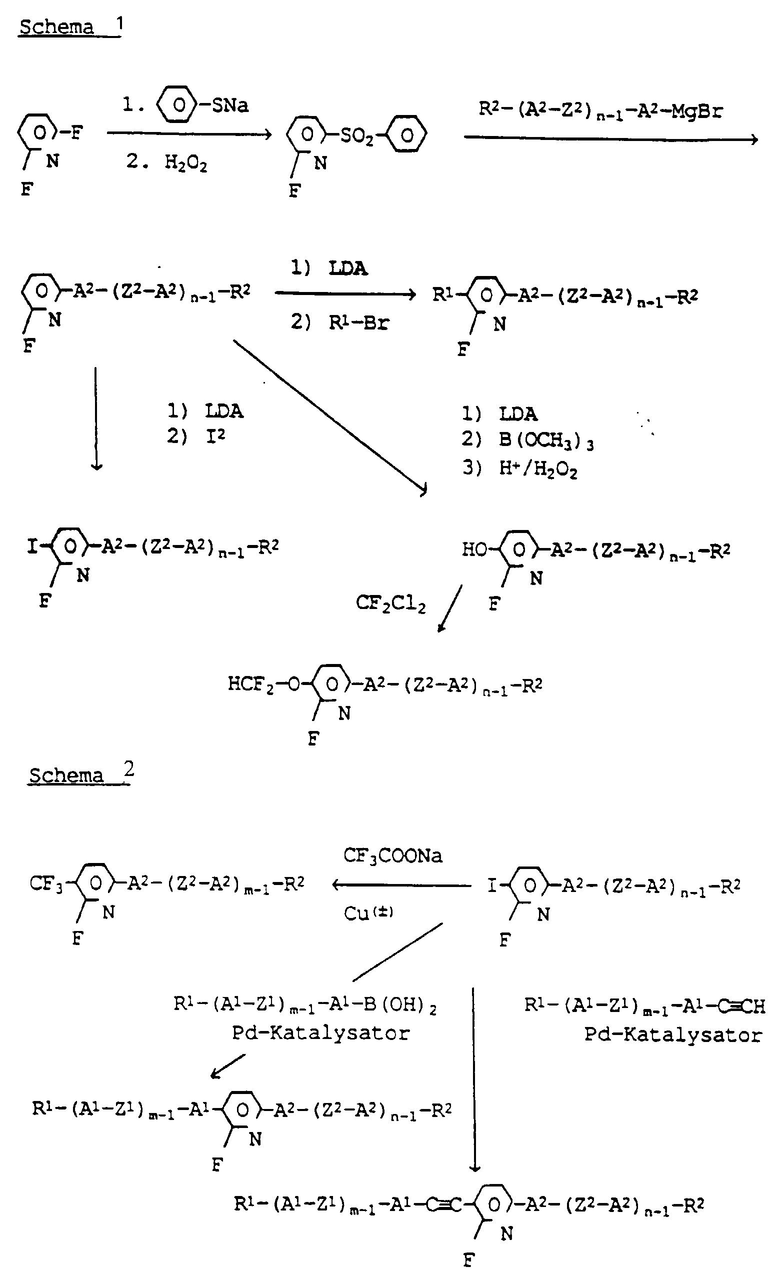

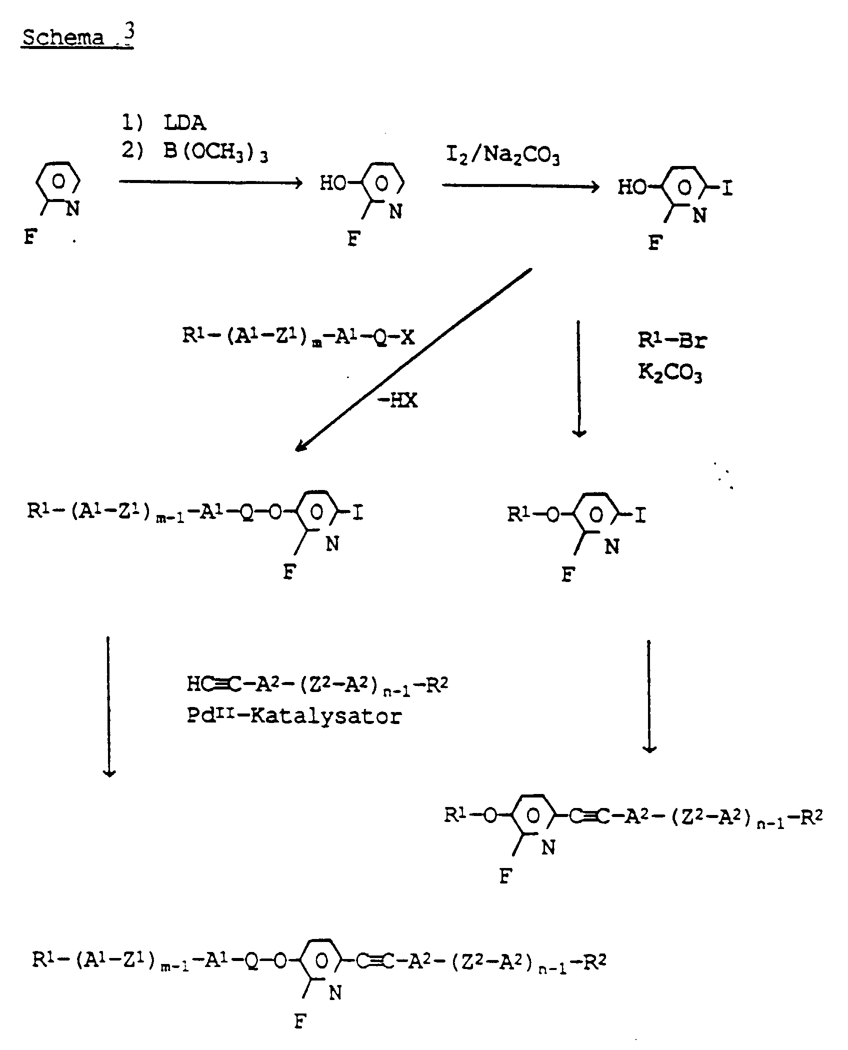

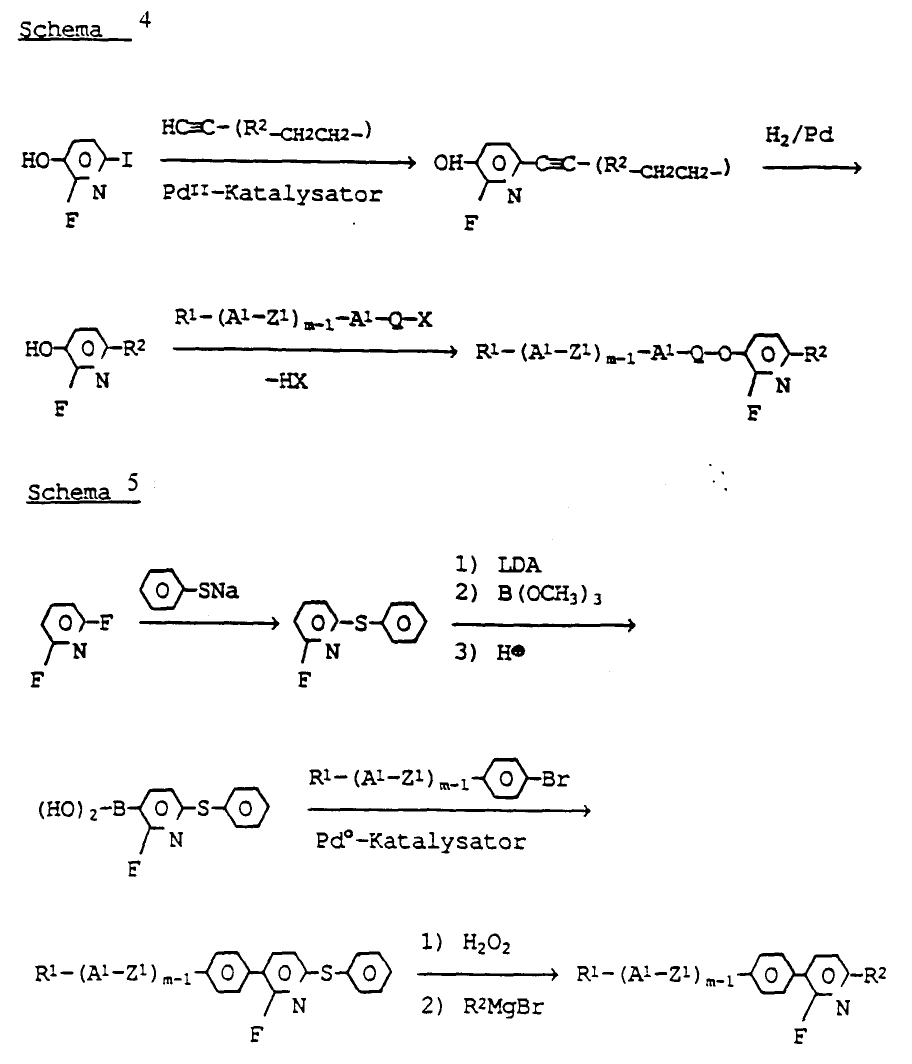

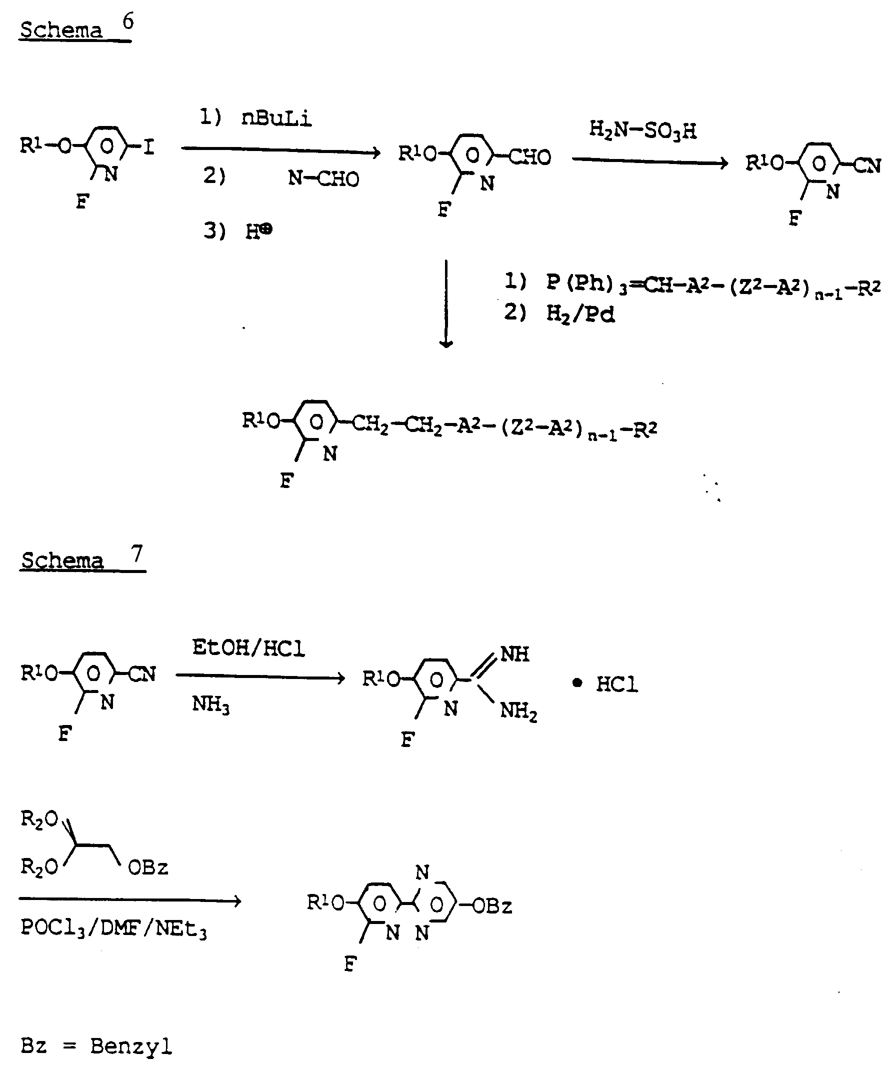

Die Benzylgruppe kann man hydrogenolytisch abspalten und dann die Hydroxygruppe erneut nach bekannten Methoden verethern oder verestern.

Die erfindungsgemäßen chiralen Verbindungen lassen sich nach folgenden Reaktionsschemata (I bis VII) herstellen.

So können zur Herstellung von Verbindungen der Formel I, worin der chirale Rest R* die Formel IIIa aufweist mit s = 2 und p = 1, geeignete Vorstufen aus optisch aktiver Äpfelsäure nach Reaktionsschema I hergestellt werden:

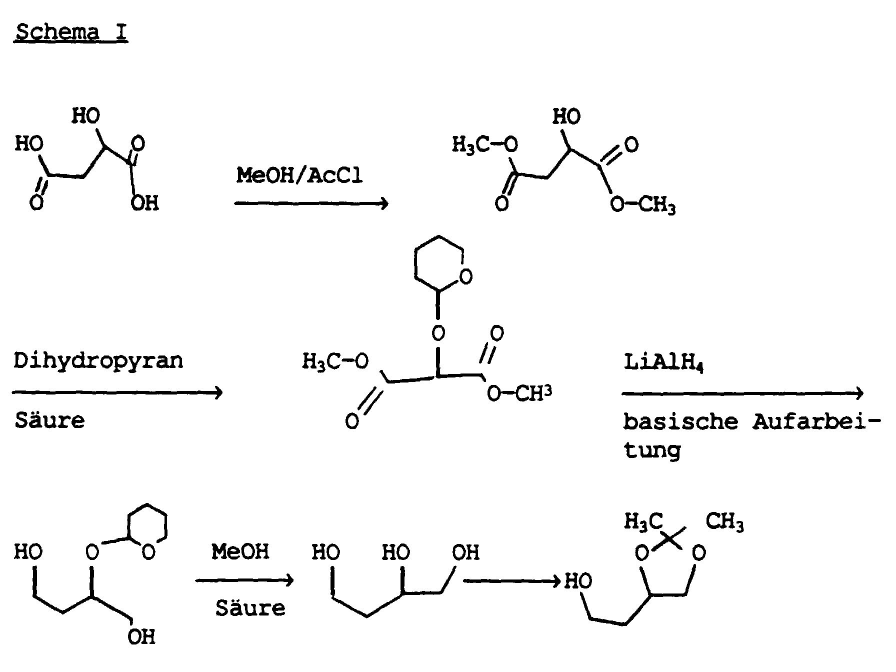

Bis zu dieser Stufe ist die Synthese von Mori et al. beschrieben worden (K. Mori, T. Takigawa and T. Matsuo, Tetrahedron 35, 933-944 (1979)).

Später haben dann Meyers and Lawson gefunden, daß die chemische Reinheit des auf diesem Weg erhaltenen Acetonids nur etwa 90 % beträgt (A.I. Meyers and J.P. Lawson, THL 23 4883-4886 (1982)).

Dessen ungeachtet kann die freie Alkoholgruppe des Acetonids nach einer der üblichen Methoden verethert werden (z.B. C.A. Brown and D. Barton, Synthesis (1974) 434 oder B.R. Jursic, Tetrahedron 44, 6677-6680 (1988)).

Der Benzylether (K. Isaac and P. Kocienski, J. Chem. Soc., Chem. Commun. (1982) 460-462) bietet sich insbesondere als Schutzgruppe an, da er später leicht hydrogenolytisch abgespalten werden kann. Unter Standardbedingungen wird nach der Veretherung das Isopropylidenketal zum 1,2-Diol hydrolisiert und dieses dann entsprechend den Reaktionsbedingungen von Di Fabio und Misiti in das entsprechende Epoxid überführt (R. Di Fabio and D. Misiti, Gazetta Chimica Italiana 118, 209-210 (1988)).

Die Behandlung des Acetonids mit HBr/Eisessig und die anschließende Umsetzung der auf diese Weise erhaltenen Bromooxalkylacetate mit K-Pentanolat liefert entsprechend der Arbeit von U. Schmidt et al. ebenfalls die gewünschten Epoxide gemäß Schema II (U. Schmidt, J. Tabiersky, F. Bartowiak and J. Wild, Angew. Chem. 92, 201-202 (1980)).

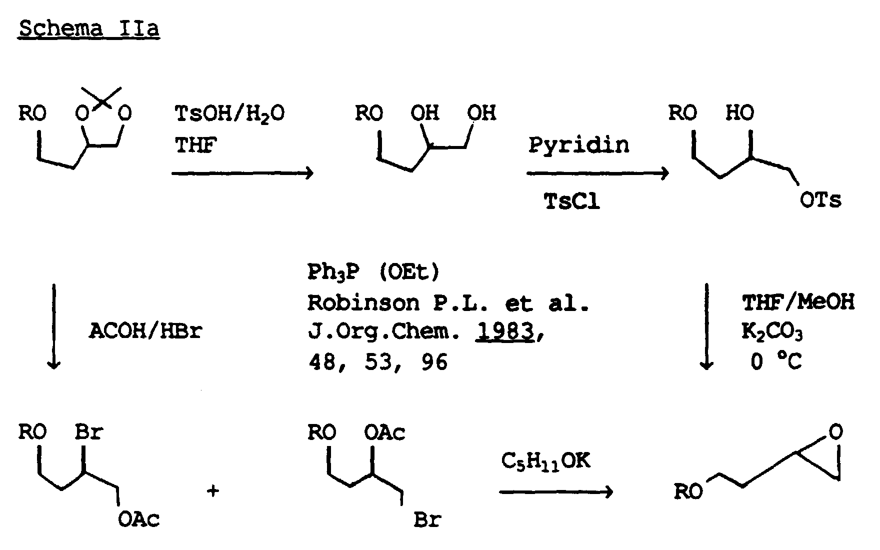

Die Umsetzung des Epoxids mit metallorganischen Verbindungen gemäß Schema IIa, bevorzugt mit Grignard-Verbindungen, ergibt unter Ringöffnung am weniger substituierten C-Atom des Epoxids mit hoher Selektivität den entsprechenden Alkohol, der mit DAST unter Standardbedingungen fluoriert wird; Hydrogenolyse liefert den chiralen Alkohol, der mit 5-Hydroxypyridinen verethert wird.

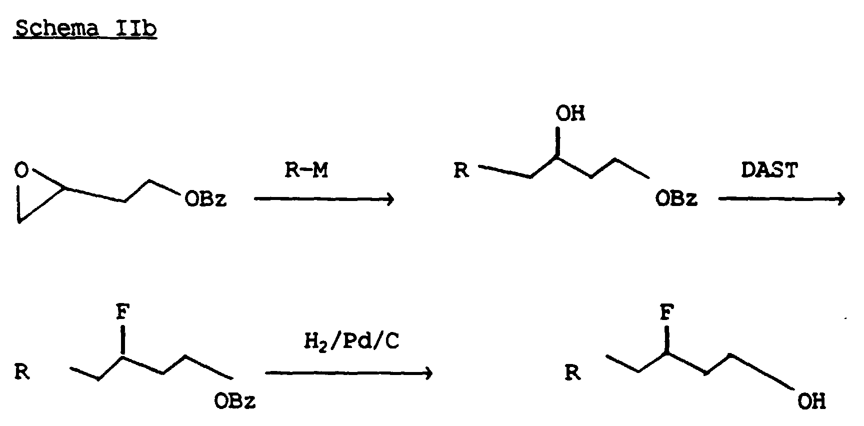

Öffnet man das Epoxid mit Pyridin/HF (N. Mongelli, F. Animati et al., Synthesis 310 (1988)), dann erhält man den entsprechenden Fluoralkohol, der anschließend in das entsprechende Tosylat überführt werden kann. Solche Tosylate eignen sich insbesondere zur Alkylierung von Phenolen und 5-Hydroxypyridinen gemäß Schema III bzw. Schema IV.

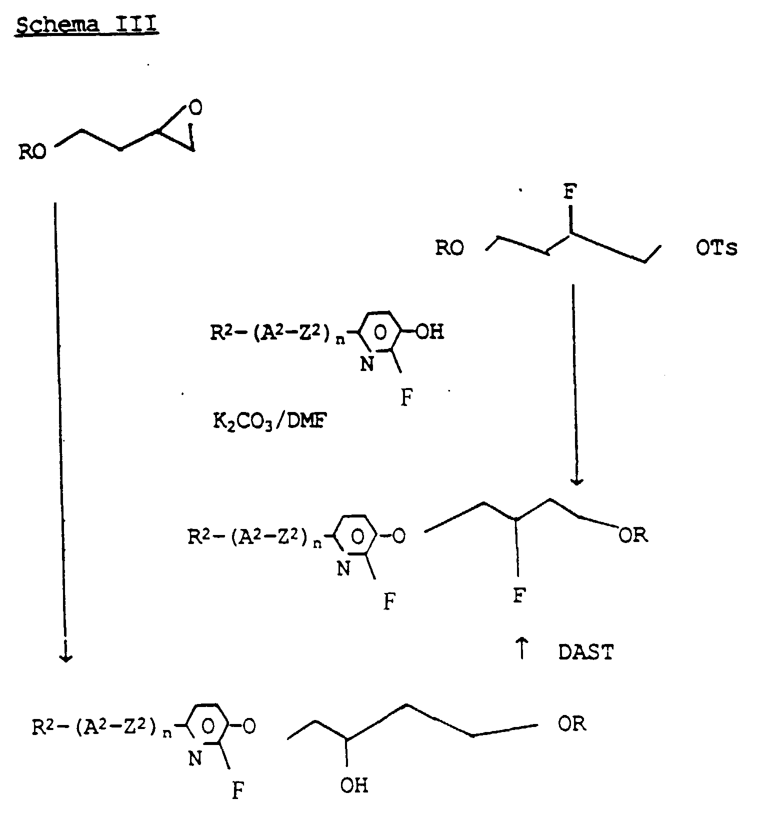

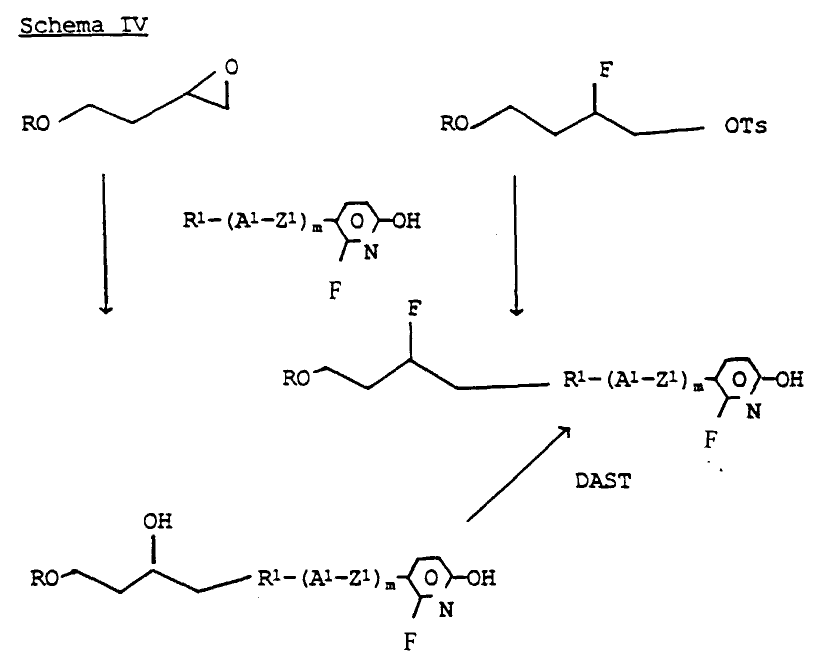

Wie obige Reaktionsschemata zeigen, kann man das Epoxid auch direkt mit Phenolen umsetzen. Das Epoxid wird mit hoher Selektivität am weniger substituierten Kohlenstoffatom zum chiralen sekundären Alkohol geöffnet, der dann schließlich mit DAST unter Inversion in die erfindungsgemäßen Verbindungen überführt wird. Zu den üblichen Umsetzungen von Alkoholen mit DAST siehe: M. Hudlicky, Organic Reactions 35 513-637 (1987).

Die erfindungsgemäßen Verbindungen mit Q³ = -O-CO- lassen sich aus den entsprechenden Benzylethern durch Hydrogenolyse und anschließende Veresterung herstellen. Folgendes Syntheseschema V beschreibt die Herstellung:

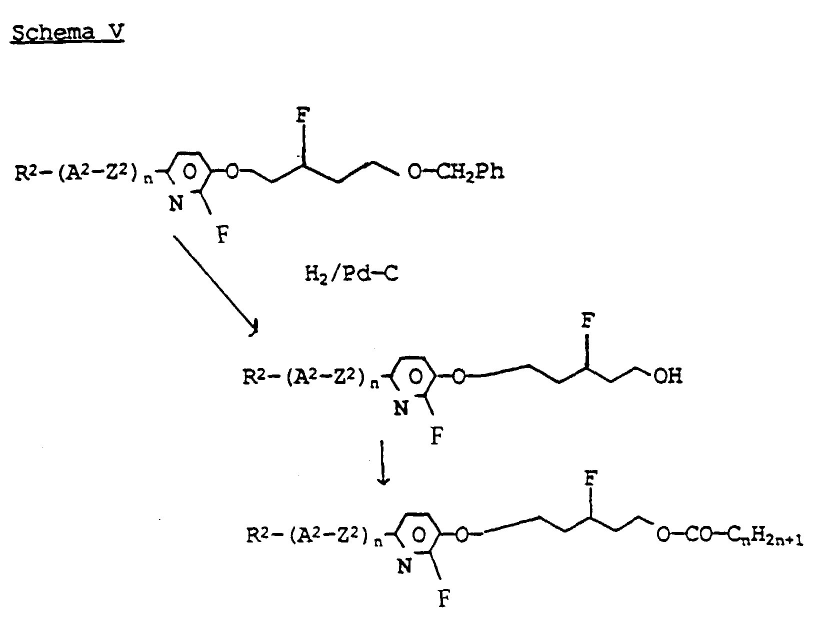

Weiterhin erhält man die Verbindungen mit Q³ = -O-CO- durch Oxidation der entsprechenden Fluoralkohole und anschließende Veresterung mit mesogenen Phenolen gemäß Schema VI:

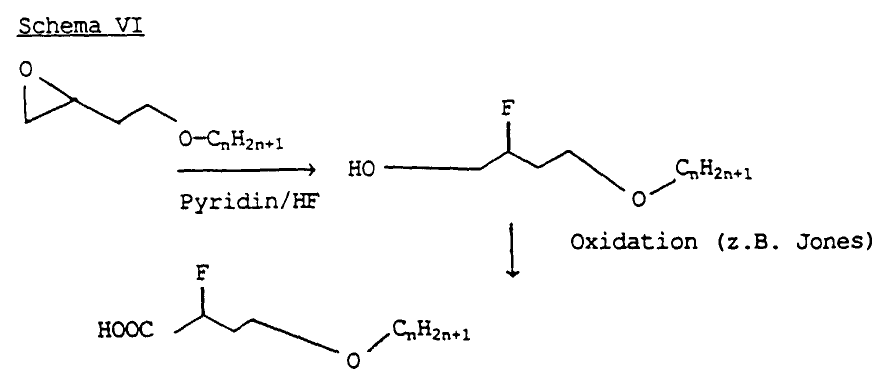

Wenn bei der Oxidation Racemisierung auftritt, kann man die optisch aktiven Fluorsäuren durch Racematspaltung nach Helmchen gewinnen (Angew. Chem. 91, 65 (1979)).

CH-azide Verbindungen öffnen in Gegenwart geeigneter Basen ebenfalls das Epoxid zum optisch aktiven sekundären Alkohol, der dann mit DAST unter Inversion fluoriert wird. Bevorzugte Reaktionswege können dem folgenden Schema VII entnommen werden.

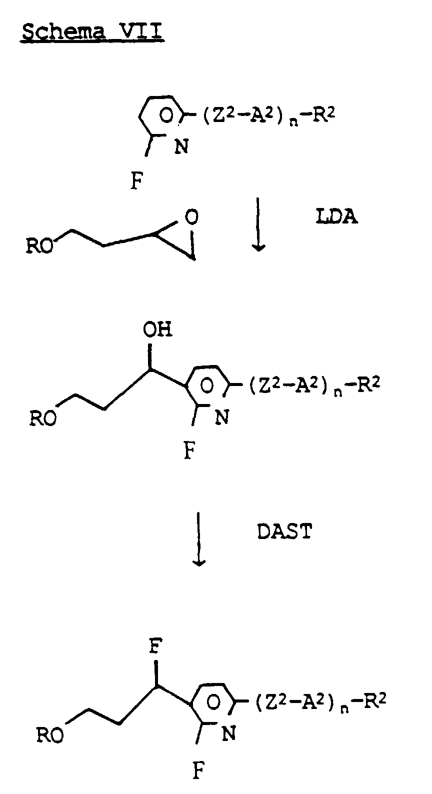

Die Verbindungen mit chiralen Resten R* der Formel IIIa, worin p 1, s 1 und Q³ O bedeuten, können analog hergestellt werden unter Einsatz der bekannten Epoxide der Formel 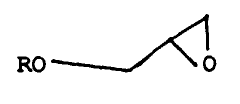 oder der aus diesen nach üblichen Verfahren erhältlichen Fluoralkohole der Formel

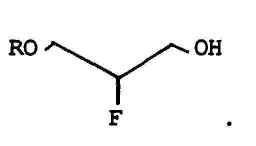

Die Verbindungen der Formel I eignen sich auch als Komponenten nematischer flüssigkristalliner Phasen, insbesondere für ECB-Anzeigen aber auch zur Vermeidung von reverse twist.

Diese erfindungsgemäßen flüssigkristallinen Phasen bestehen aus 2 bis 25, vorzugsweise 3 bis 15 Komponenten, darunter mindestens einer Verbindung der Formel I. Die anderen Bestandteile werden vorzugsweise ausgewählt aus den nematischen oder nematogenen Substanzen, insbesondere den bekannten Substanzen, aus den Klassen der Azoxybenzole, Benzylidenaniline, Biphenyle, Terphenyle, Phenyl- oder Cyclohexylbenzoate, Cyclohexan-carbonsäurephenyl- oder cyclohexylester, Phenylcyclohexane, Cyclohexylbiphenyle, Cyclohexylcyclohexane, Cyclohexylnaphthaline, 1,4-Bis-cyclohexylbenzole, 4,4'-Bis-cyclohexylbiphenyle, Phenyl- oder Cyclohexylpyrimidine, Phenyl- oder Cyclohexylpyridazine sowie deren N-Oxide, Phenyl- oder Cyclohexyldioxane, Phenyl- oder Cyclohexyl-1,3-dithiane, 1,2-Diphenylethane, 1,2-Dicyclohexylethane, 1-Phenyl-2-cyclohexylethane, gegebenenfalls halogenierten Stilbenen, Benzylphenylether, Tolane und substituierten Zimtsäuren.

Die wichtigsten als Bestandteile derartiger flüssigkristalliner Phasen in Frage kommenden Verbindungen lassen sich durch die Formel I'' charakterisieren,

R'-L-G-E-R'' I''

worin L und E je ein carbo- oder heterocyclisches Ringsystem aus der aus 1,4-disubstituierten Benzol- und Cyclohexanringen, 4,4'-disubstituierten Biphenyl-, Phenylcyclohexan- und Cyclohexylcyclohexansystemen, 2,5-disubstituierten Pyrimidin- und 1,3-Dioxanringen, 2,6-disubstituiertem Naphthalin, Di- und Tetrahydronaphthalin, Chinazolin und Tetrahydrochinazolin gebildeten Gruppe,

| | | |
|---|---|---|
| G | -CH=CH- | -N(0)=N- |
| | -CH=CY- | -CH=N(0)- |
| | -C≡C- | -CH₂-CH₂- |
| | -C0-0- | -CH₂-0- |
| | -C0-S- | -CH₂-S- |
| | -CH=N- | -C00-Phe-C00- |

oder eine C-C-Einfachbindung,
Y Halogen, vorzugsweise Chlor, oder -CN, und R' und R'' Alkyl, Alkoxy, Alkanoyloxy, Alkoxycarbonyl oder Alkoxycarbonyloxy mit bis zu 18, vorzugsweise bis zu 8 Kohlenstoffatomen, oder einer dieser Reste auch CN, NC, N0₂, CF₃, F, Cl oder Br bedeuten.

Bei den meisten dieser Verbindungen sind R' und R'' voneinander verschieden, wobei einer dieser Reste meist eine Alkyl- oder Alkoxygruppe ist. Aber auch andere Varianten der vorgesehenen Substituenten sind gebräuchlich. Viele solcher Substanzen oder auch Gemische davon sind im Handel erhältlich. Alle diese Substanzen sind nach literaturbekannten Methoden erhältlich.

Die erfindungsgemäßen Phasen enthalten etwa 0,1 bis 99, vorzugsweise 10 bis 95 %, einer oder mehrerer Verbindungen der Formel I. Weiterhin bevorzugt sind erfindungsgemäße flüssigkristalline Phasen, enthaltend 0,1-40, vorzugsweise 0,5-30 % einer oder mehrerer Verbindungen der Formel I. Die Herstellung der erfindungsgemäßen Phasen erfolgt in an sich üblicher Weise. In der Regel werden die Komponenten ineinander gelöst, zweckmäßig bei erhöhter Temperatur. Durch geeignete Zusätze können die flüssigkristallinen Phasen nach der Erfindung so modifiziert werden, daß sie in allen bisher bekannt gewordenen Arten von Flüssigkristallanzeigeelementen verwendet werden können.

Derartige Zusätze sind dem Fachmann bekannt und in der Literatur ausführlich beschrieben. Beispielsweise können Leitsalze, vorzugsweise Ethyldimethyldodecylammonium-4-hexyloxybenzoat, Tetrabutylammonium-tetraphenylboranat oder Komplexsalze von Kronenethern (vgl. z.B. I. Haller et al., Mol. Cryst.Liq.Cryst. Band 24, Seiten 249-258 (1973)) zur Verbesserung der Leitfähigkeit, pleochroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden.

Derartige Substanzen sind z.B. in den DE-OS 22 09 127, 22 40 864, 23 21 632, 23 38 281, 24 50 088, 26 37 430, 28 53 728 und 29 02 177 beschrieben.

Die beschriebenen Mischungen eignen sich in besonderem Maße als Komponenten für Flüssigkristall-Schalt- und Anzeigevorrichtungen, insbesondere für die eingangs beschriebene SSFLC-Zelle. Insbesondere gilt dies für die sogenannte "jungfräuliche (virgin) Flüssigkristall-Textur" bei der die smektischen Lagen gewinkelt verlaufen ("Chevron-Strukturen") und für die "bookshelf"- oder "quasi-bookshelf"-Geometrie, bei denen die smektischen Lagen senkrecht oder nahezu senkrecht verlaufen (s. Y. Sato et al., Jap. J. Appl. Phys. Bd. 28, 483 (1989)).

Daneben eignen sich die nematischen Mischungen enthaltend Verbindungen der Formel I aufgrund ihrer breiten nematischen Phasenbereiche und ihrer hohen negativen dielektrischen Anisotropie in besonderen Maße als Komponenten für höher verdrillte Flüssigkristallanzeigen wie STN (z.B. GB 2,123,163) oder OMI (z.B. M. Schadt, F. Leenhouts, Appl. Phys. Lett. 50, 236 (1987)), wobei sehr hohe Steilheiten der Kennlinie erzielt werden können (z.B. WO 89/08691).

Insbesondere die Verbindungen der Formel IT eignen sich hervorragend als Komponenten für ECB-Anzeigen (z.B. M. Schiekel, K. Fahrenschon, Appl. Phys. Lett. 19 391 (1971)), das sie sehr große Werte der optischen Anisotropie (Δn ≥ 0,22) und vergleichsweise hohe negative Werte der dielektrischen Anisotropie aufweisen (Δε ≤ -5,0).

Die folgenden Beispiele sollen die Erfindung erläutern, ohne sie zu begrenzen. F. = Schmelzpunkt, K. = Klärpunkt. Vor- und nachstehend bedeuten Prozentangaben Gewichtsprozent; alle Temperaturen sind in Grad Celsius angegeben. "Übliche Aufarbeitung" bedeutet: man gibt Wasser hinzu, extrahiert mit Methylenchlorid, trennt ab, trocknet die organische Phase, dampft ein und reinigt das Produkt durch Kristallisation und/oder Chromatographie.

Weiterhin bedeuten:
- K: kristallin
- N: nematisch
- S: smektisch
- I: isotrop

Die zwischen diesen Symbolen stehenden Zahlen geben die jeweilige Phasenübergangstemperatur in °C an. Ferner werden folgende Abkürzungen verwendet:
- n-BuLi: n-Butyllithium
- DAST: Diethylaminoschwefeltrifluorid
- DCC: Dicyclohexylcarbodiimid
- DEAD: Diethylazodicarboxylat
- DMF: Dimethylformamid
- LDA: Lithiandiisopropylamid (hergestellt aus equimolaren Mengen von n-BuLi und Diisopropylamin)
- THF: Tetrahydrofuran
- TP: Triphenylphosphin
- TPP: Tetrakis(triphenylphosphin)palladium (O)

### Beispiel 1

### 1A 2-Fluor-6-phenylsulfonylpyridin

Ein Gemisch aus 0,4 mol 2,6-Difluorpyridin, 0,6 mol Thiophenol, 0,6 mol NaOH, 450 ml Wasser, 180 ml Toluol und 3,5 g Tetrabutylammoniumbromid wird 4 Stunden unter Rückfluß erhitzt.

Nach Abkühlen auf Raumtemperatur wird die organische Phase abgetrennt und die wäßrige mit Hexan extrahiert. Die vereinten organischen Phasen werden mit einer gesätigten Kochsalzlösung gewaschen und mit Natriumsulfat getrocknet. Nach Eindampfen des Lösungsmittels wird der Rückstand mit 22 g einer 35%igen H₂O₂-Lösung und 50 ml Eisessig versetzt und 18 Stunden zum Sieden erhitzt. Nach Abkühlen, üblichem Aufarbeiten und Chromatographie über Kieselgel mit Dichlormethan erhält man das reine Produkt.

### 1B 2-(4-Octyloxyphenyl)-6-fluorpyridin

Ein Gemisch aus 0,06 mol 4-Octyloxyphenylmagnesiumbromid (hergestellt aus 0,06 mol 4-Octyloxybrombenzhol und 0,06 mol 4-Octyloxybrombenzol und 0,06 mol Magnesium) und 25 ml THF wird bei 30-40 °C zu einem Gemisch aus 0,05 mol 2A und 15 ml THF gegeben. Nach 2stündigem Rühren bei Raumtemperatur wird wie üblich aufgearbeit. Das ungereinigte Produkt wird nach 2C weiterverarbeitet.

### 1C 2-(4-Octyloxyphenyl)-5-hydroxy-6-fluorpyridin

0,05 mol 2B, 0,08 mol LDA, 0,08 mol Trimethylborat, und 0,16 mol H₂O₂ (in Form einer 30%igen Lösung) erhält man analog Beispiel 1B das Produkt.

### 1D 2-(Octyloxyphenyl)-5-octyloxy-6-fluorpyridin

Aus 20 mmol 2C, 20 mmol Octanol, 20 mmol TP und 20 mmol DEAD erhält man analog Beispiel 1C das Produkt, K 81 S_{C} 89 I, Δε = -4,3, Δn = 0,161

Analog werden hergestellt:

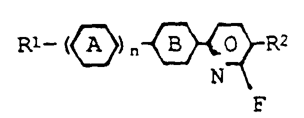

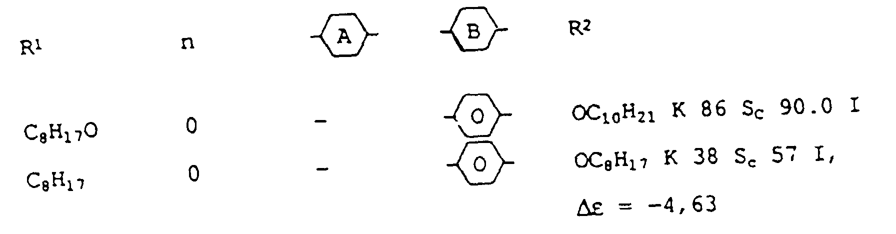

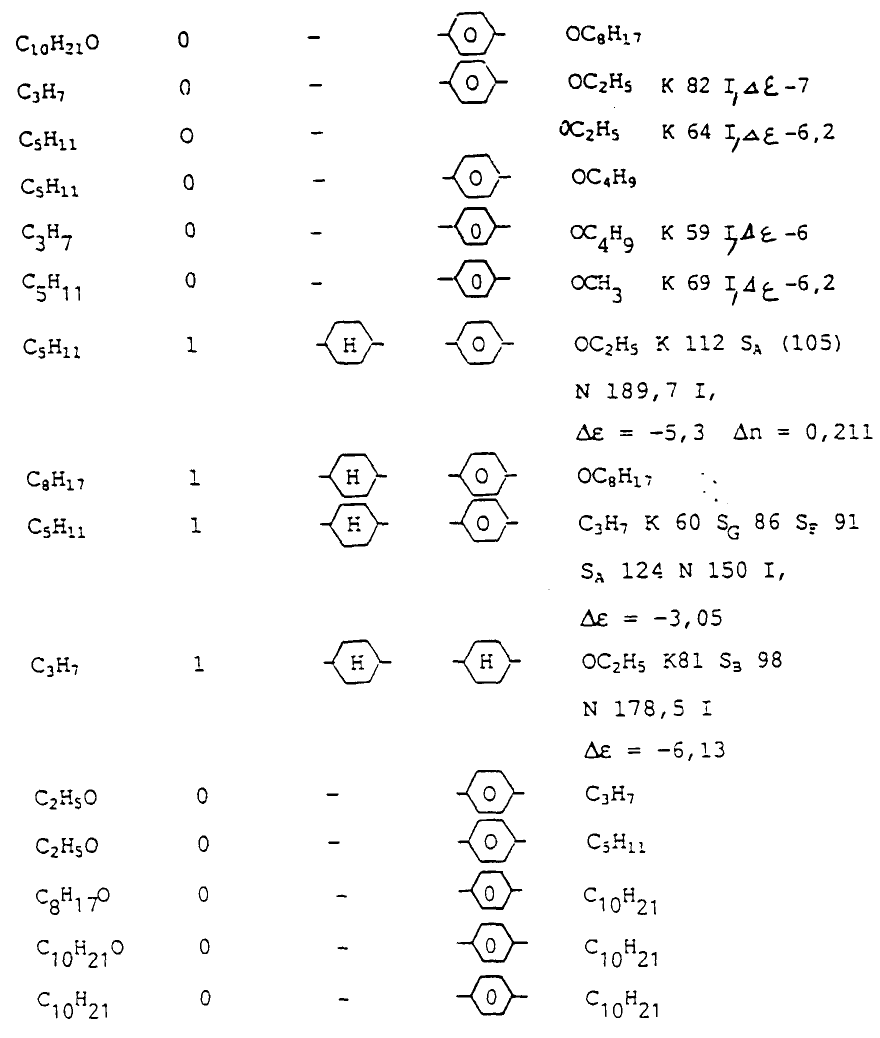

### Beispiel 2

Man kühlt eine Lösung von 0,1 mol 2-(Octylphenyl)-5-(2-hydroxy-5-oxaoctyloxy)-6-fluorpyridin (hergestellt durch Erhitzen von optisch aktivem 1,2-Epoxy-5-oxaoctan, erhältlich aus Äpfelsäure, mit 2C in Gegenwart von trockenem Kaliumcarbonat und Methylethylketon als Lösungsmittel) in Methylenchlorid auf -40 °C und gibt dazu unter Feuchtigkeitsausschluß tropfenweise 0,11 mol DAST. Anschließend rührt man das Reaktionsgemisch unter langsamem Erwärmen auf Raumtemperatur 12 Stunden. Dann hydrolysiert man unter Eiskühlung und wäscht das Reaktionsgemisch mit verdünnter Natronlauge und mehrfach mit Wasser. Nach dem Trocknen über Magnesiumsulfat wird das Lösungsmittel am Rotationsverdampfer entfernt und das Rohprodukt chromatographisch und durch Kristallisation gereinigt. Man erhält optisch aktives 2-(4-Octyloxyphenyl)-5-(2-fluor-5-oxaoctyloxy)-6-fluorpyridin.

Analog werden folgende Verbindungen der Formel I1 hergestellt:

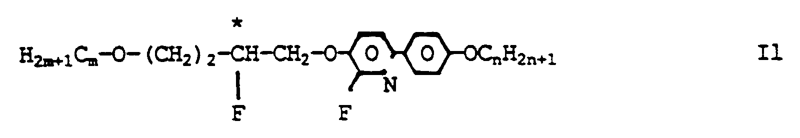

| m | n |
|---|---|
| 2 | 7 |
| 4 | 7 |
| 5 | 7 |
| 2 | 8 |
| 3 | 8 |
| 4 | 8 |
| 5 | 8 |
| 3 | 9 |
| 3 | 10 |

### Beispiel 3

Aus 0,1 mol 4-(6-Fluor-5-octyloxypyridin-2-yl)-phenol (hergestellt entsprechend Beispiel 2 durch Hydrolyse von 4-(6-Fluor-5-octyloxypyridin-2-yl)-1-benzyloxybenzol) und 0,1 mol optisch aktiven 1,2-Epoxy-5-octan erhält man in Analogie zu Beispiel 3 4-(6-Fluor-5-octyloxypyridin-2-yl)-1-(2-fluor-5-oxyoctyl)-benzol.

### Analog werden folgende Verbindungen der Formel I1 hergestellt:

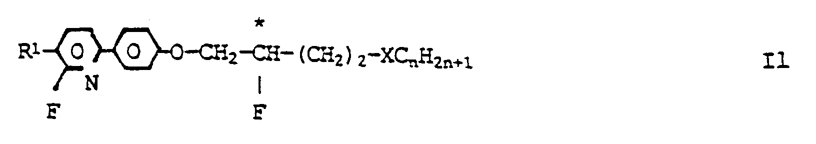

| R¹ | n | X |
|---|---|---|
| C₇H₁₅O | 3 | O |
| C₉H₁₉O | 3 | O |
| C₁₁H₂₃O | 3 | O |
| C₈H₁₇ | 3 | CH₂ |

### Beispiel 4

4-(6-Fluor-5-(2-fluor-5-oxyoctyl)pyridin-2-yl)-1-octylbenzol 0,1 mol 2C (hergestellt nach Beispiel 1) und 0,1 mol optisch aktives 2-Fluoroctanol-1 werden in 200 ml THF gelöst und bei RT mit 0,1 m Triphenylphosphin und 0,1 m DEAD versetzt. Man rührt noch 2^{h} bei RT und arbeitet extraktiv auf:

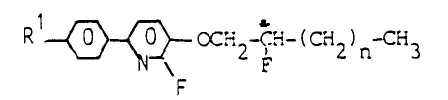

| R¹ | n | R¹ | n |
|---|---|---|---|
| C₈H₁₇ | 5 K 80 I | | |
| C₇H₁₅0 | 5 | C₇H₁₅ | 5 |
| C₈H₁₇O | 5 | C₈H₁₇ | 5 |
| C₉H₁₉O | 5 | C₉H₁₉ | 5 |
| C₁₀H₂₁O | 5 | C₁₀H₂₁ | 5 |

### Beispiel 5

Zu einer Lösung von 0,15 mol 2C (hergestellt entsprechend Beispiel 1), 0,17 mol L(-)-Ethyllactat und 0,15 mol Triphenylphosphin in 400 ml THF gibt man 0,17 mol DEAD gelöst in THF. Dabei soll eine Reaktionstemperatur von 50 °C nicht überschritten werden. Man rührt 1 Stunde bei 50 °C und dann über Nacht bei Raumtemperatur. Anschließend destilliert man das Lösungsmittel ab, löst den Rückstand in heißem Toluol und läßt dann langsam abkühlen. Das ausgefallene Triphenylphosphinoxid wird abgesaugt, das Filtrat eingeengt und der Rückstand chromatographisch gereinigt. Man erhält 2-[2-(4-Oc-tyloxyphenyl)-6-fluorpyridin-5-yl]-propionsäureethylester.

### Beispiel 6

Optisch aktiver Milchsäurebenzylester wird mittels Diethylazodicarboxylat (DEAD)/Triphenylphosphin mit 2C (hergestellt entsprechend Beispiel 1) verethert und anschließend die Benzylgruppe hydrogenolytisch abgespalten. Die so gewonnene Säure wird wie üblich in das Nitril überführt (Oxalylchlorid, Ammoniak, Thionylchlorid). Man erhält optisch aktives 2-(4-Octyloxyphenyl)-5-(1-cyanoethoxy)-6-fluorpyridin.

### Beispiel 7

Zu einem Gemisch aus 0,1 mol 2C 0,1 mol optisch aktiver 2-Chlor-3-methylbuttersäure (hergestellt aus Valin) und einer katalytischen Menge 4-(N,N-Dimethylamino)pyridin in 250 ml Methylenchlorid gibt man bei 0 °C eine Lösung von 0,1 mol DCC in Methylenchlorid. Anschließend läßt man 12 Stunden bei Raumtemperatur ruhen, saugt dann den Niederschlag ab, arbeitet das Filtrat wie üblich auf und erhält [2-(4-Octyloxyphenyl)-6-fluorpyridin-5-yl-ester].

### Beispiel 8

Zu einem Gemisch aus 0,01 mol 5-Ethoxy-2-jod-6-fluorpyridin (hergestellt aus 2-Fluorpyridin durch Umsetzung mit Lithiumdiispropylamid, Triethylborat und Wasserstoffperoxid, Jodierung des so erhaltenen 3-Hydroxy-2-fluorpyridins und anschließender Veretherung mit Jodethan/Kaliumcarbonat), 0,01 mol 4-(trans-4-Propylcyclohexyl)-phenylacetylen (herstellbar z.B. nach Smith, Hoehn, Am. Soc. 63 (1941) 117) und 40 ml Triethylamin gibt man bei Raumtemperatur 0,2 mmol Bis(triphenylphosphin)-palladium(II)-chlorid und 0,1 mmol Kupfer(I)-jodid und rührt 12 Stunden. Die Reaktion läßt sich mit Hilfe der Dünnschichtchromatographie verfolgen. Nach beendeter Reaktion wird die Suspension filtriert und das Filtrat eingedampft. Nach Reinigung durch Chromatographie und/oder Kristallisation erhält man 1-(5-Ethoxy-6-fluorpyridin-2-yl)-1-[4-(trans-4-propylcyclohexyl)-phenyl]-acetylen.

Analog werden hergestellt:

1-(5-Ethoxy-6-fluorpyridin-2-yl)-1-(4-pentylphenyl)-acetylen, K 69 N (54,1) I Δε = -7,83, Δn = 0,252.

### Beispiel 9

Ein Gemisch von 0,1 mol 2C, 0,1 mol 1-Bromethoxyethan, 0,1 mol Natriumhydroxid, 20 ml Ethanol und 50 ml Wasser wird 2 Stunden zum Sieden erhitzt. Nach Abkühlen wird das Gemisch mit verdünnter Salzsäure versetzt und die wäßrige Phase mit Dichlormethan extrahiert. Nach Entfernen des Lösungsmtitels unter vermindertem Druck wird der Rückstand durch Säulenchromatographie gereinigt, man erhält das Produkt

2-(4-Octyloxyphenyl)-5-(2-ethoxyethoxy)-6-fluorpyridin

Analog werden hergestellt:

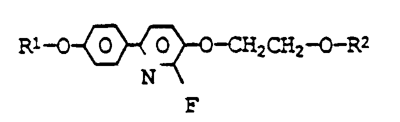

| R¹ | R² |
|---|---|
| C₅H₁₁ | C₂H₅ |
| C₈H₁₇ | C₄H₉ |
| C₈H₁₁ | CH₃ |
| C₈H₁₇ | C₂H₄-O-C2H₅ |
| C₅H₁₁ | C₂H₄-O-C2H₅ |
| C₁₁H₂₃ | C₂H₄-O-C2H₅ |

Die folgenden Beispiele betreffen ferroelektrische flüssigkristalline Medien.

### Beispiel A

Zu einer achiralen Basismischung bestehend aus
- 3,3 %: 2-(4-Hexyloxyphenyl)-5-heptylpyrimidin
- 3,3 %: 2-(4-Heptyloxyphenyl)-5-heptylpyrimidin
- 3,3 %: 2-(4-Octyloxyphenyl)-5-heptylpyrimidin
- 3,3 %: 2-(4-Nonyloxyphenyl)-5-heptylpyrimidin
- 7,7 %: 2-(4-Hexyloxyphenyl)-5-nonylpyrimidin
- 25,3 %: 2-(4-Nonyloxyphenyl)-5-nonylpyrimidin
- 30,8 %: 4-(4'-Octylbiphenyl-4-yl)-1-cyano-1-butylcyclohexan
- 15,4 %: 4-(4'-Heptylbiphenyl-4-yl)-1-cyano-1-hexylcyclohexan
- 6,6 %: 1,4-Bis-[trans-4-pentylcyclohexyl]-1-cyanocyclohexan
welche folgende Phasenübergänge aufweist:
S_{C} 76 S_{A} 80 N 36 I
werden 10 % des chiralen 2-(4-Octylphenyl)-5-(2-fluoroctyloxy)-6-fluorpyridin gegeben.

Das ferroelektrische Medium zeigt:
S_{C}* 73 S_{A} 78 CN 91 I

Spontanpolarisation (bei 20 °C): 17,6 nC·cm⁻¹
Schaltzeit (bei 20 °C und 15 V/µm): 85 µsec

### Beispiel 10

### 10A 2-Propin-1-yl-6-fluorpyridin

Ein Gemisch aus 0,06 mol Propinylmagnesiumbromid und 25 ml THF wird bei -40-40 °C zu einem Gemisch aus 0,05 mol 2A und 15 ml THF gegeben. Nach 2stündigem Rühren bei Raumtemperatur wird wie üblich aufgearbeitet. Das ungereinigte Produkt wird nach 10B weiterverarbeitet.

### 10B 2-Propinyl-6-fluorpyridin-5-ylboronsäure

0,05 mol 10A, 0,08 mol LDA und 0,08 mol Trimethylborat werden analog Beispiel 1B miteinander umgesetzt. Nach wäßriger Aufarbeitung erhält man das ungereinigte Produkt, das nach 10C weiterverarbeitet wird.

### 10C 2-Propyl-6-fluor-5-[4-(trans-4-pentylcyclohexyl)-phenyl]-pyridin

0,02 mol 10B werden mit 0,02 mol 4-(trans-4-Pentylcyclohexyl)-brombenzol entsprechend EP 0 354 434 in Gegenwart von Natriumhydrogencarbonat und Tetrakis[triphenylphosphin]palladium gekoppelt. Nach üblicher Aufarbeitung und chromatographischer Reinigung erhält man das Alkin-Zwischenprodukt als farblosen Feststoff, welches mit Pd-Kohle in THF zur Alkylverbindung hydriert wird, K 66 N 123 I.

Analog werden hergestellt:

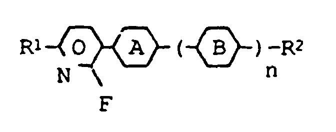

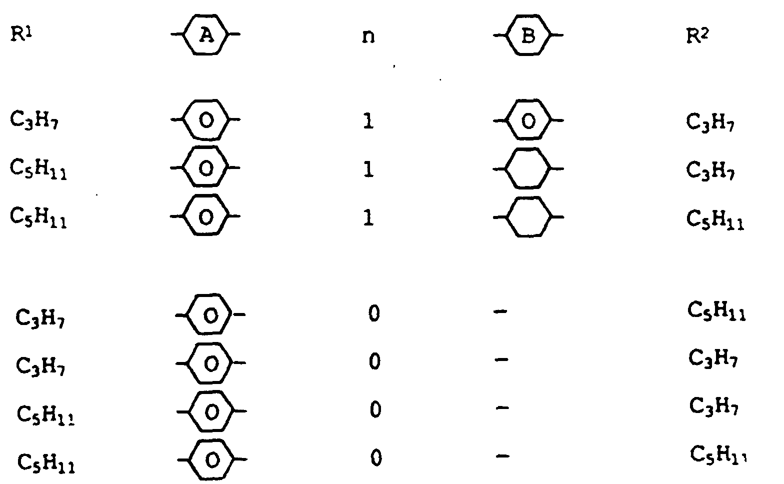

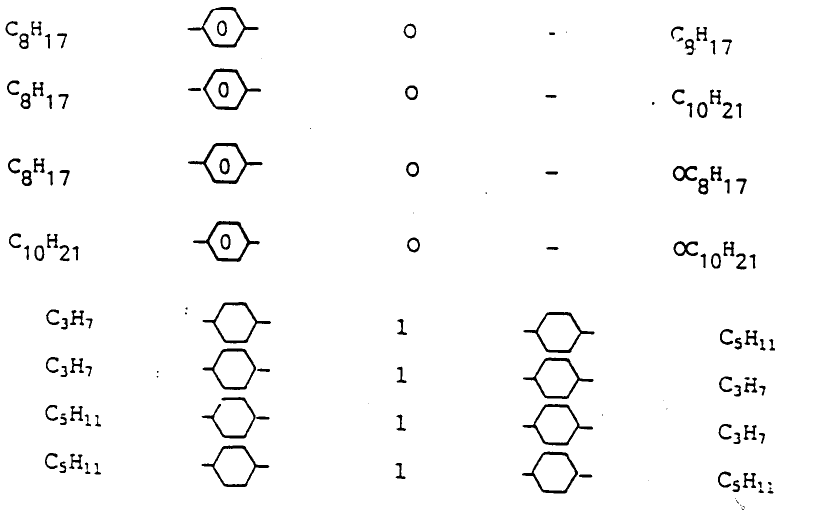

### Beispiel B

Man stellt ein flüssigkristallines Medium her bestehend aus:
- 20,00 %: 2-(p-Heptyloxyphenyl)-5-nonylpyrimidin
- 20,00 %: 2-(p-Octyloxyphenyl)-5-nonylpyrimidin
- 20,00 %: 2-(p-Nonyloxphenyl)-5-nonylpyrimidin
- 20,00 %: 2-(p-Hexyloxyphenyl)-5-hexyloxypyrimidin
- 6,00 %: 2-(p-Octyloxyphenyl)-6-fluor-5-octyloxypyrimidin
- 6,00 %: 2-(p-Octyloxyphenyl)-6-fluor-5-decyloxpyrimidin
- 6,66 %: 2-(p-Octyloxyphenyl)-6-fluor-5-octyloxypyrimidin

Dieses Medium weist folgende Phasenübergänge auf K 5 S_{C} 69 CM 74 I.

### Beispiel C

Man stellt ein ferroelektrisches, flüssigkristallines Medium her bestehend aus:
- 19,00 %: 2-(p-Heptyloxyphenyl)-5-nonylpyrimidn
- 19,00 %: 2-(p-Octyloxyphenyl)-5-nonylpyrimidn
- 19,00 %: 2-(p-Nonyloxyphenyl)-5-nonylpyrimidn
- 19,00 %: 2-(p-Hexyloxyphenyl)-5-hexyloxypyrimidn
- 6,34 %: 2-(4-Heptyloxy-2,3-difluorphenyl)-5-nonylpyrimidin
- 6,34 %: 2-(4-Octyloxy-2,3-difluorphenyl)-5-nonylpyrimidin
- 6,33 %: 2-(4-Nonyloxy-2,3-difluorphenyl)-5-nonylpyrimidin
- 5,00 %: 2-(p-Octylphenyl)-6-fluor-5-(2-fluoroctyloxy)-pyridin

Dieses Medium weist folgende physikalischen Eigenschaften auf:
K 5 S_{C}* 63 S_{A} 67 Ch 71 I
Pₛ (20 °C): 6,8 nC·cm⁻²
τ (20 °C/15 Vµm⁻¹): 84 µs

### Beispiel D

Man stellt ein nematisches flüssigkristallines ECB-Medium her bestehend aus:
- 9,0 %: p-(trans-4-Propylcyclohexyl)-methoxybenzol
- 9,0 %: p-(trans-4-Propylcyclohexyl)-ethoxybenzol
- 9,0 %: p-(trans-4-Propylcyclohexyl)-butoxybenzol
- 5,0 %: 4-Ethyl-4'-methoxytolan
- 5,0 %: 4-Methyl-4'-ethoxytolan
- 4,0 %: 2,2',3,3'-Tetrafluor-4-butoxy-4'-propyltolan
- 4,0 %: 2,2',3,3'-Tetrafluor-4-butoxy-4'-pentyltolan
- 13,0 %: 2,3-Difluor-4-ethoxy-4'-propyltolan
- 13,0 %: 2,3-Difluor-4-ethoxy-4'-pentyltolan
- 5,0 %: 4-(trans-4-Propylcyclohexyl)-4'-methoxytolan
- 5,0 %: 4-(trans-4-Propylcyclohexyl)-4'-ethoxytolan
- 5,0 %: 4-(trans-4-Propylcyclohexyl)-4'-propoxytolan
- 2,0 %: trans,trans-4-Propylcyclohexylcyclohexan-4'-carbonsäure-(2,3-difluor-4-ethoxyphenylester)
- 7,0 %: 2-(trans,trans-4-Propylcycohexylcyclohexan-4'-yl)-6-fluor-5-ethoxypyridin
- 5,0 %: 1-(p-Pentylphenyl)-2-(6-fluor-5-ethoxypyridin-2-yl)-ethin

### Beispiel E

Man stellt ein nematisches flüssigkristallines ECB-Medium her bestehend aus:
- 9,0 %: p-(trans-4-Propylcyclohexyl)-methoxybenzol
- 9,0 %: p-(trans-4-Propylcyclohexyl)-ethoxybenzol
- 9,0 %: p-(trans-4-Propylcyclohexyl)-butoxybenzol
- 5,0 %: 4-Ethyl-4'-methoxytolan
- 5,0 %: 4-Methyl-4'-ethoxytolan
- 4,0 %: 2,2',3,3'-Tetrafluor-4-butoxy-4'-propyltolan
- 4,0 %: 2,2',3,3'-Tetrafluor-4-butoxy-4'-pentyltolan
- 13,0 %: 2,3-Difluor-4-ethoxy-4'-propyltolan
- 13,0 %: 2,3-Difluor-4-ethoxy-4'-pentyltolan
- 5,0 %: 4-(trans-4-Propylcyclohexyl)-4'-methoxytolan
- 5,0 %: 4-(trans-4-Propylcyclohexyl)-4'-ethoxytolan
- 5,0 %: 4-(trans-4-Propylcyclohexyl)-4'-propoxytolan
- 6,0 %: 4-(trans,trans-4-Propylcyclohexylcyclohexan-4'-yl)-2,3-difluor-ethoxybenzol
- 5,0 %: 4'-(trans-4-Pentylcyclohexyl)-2,3-difluor-4-ethoxybiphenyl
- 3,0 %: 2-(p-Pentylphenyl)-6-fluor-5-ethoxypyridin

### Beispiel F

Man stellt ein nematisches flüssigkristallines ECB-Medium her bestehend aus:
- 9,0 %: p-(trans-4-Propylcyclohexyl)-methoxybenzol
- 9,0 %: p-(trans-4-Propylcyclohexyl)-ethoxybenzol.
- 9,0 %: p-(trans-4-Propylcyclohexyl)-butoxybenzol
- 5,0 %: 4-Ethyl-4'-methoxytolan
- 5,0 %: 4-Methyl-4'-ethoxytolan
- 4,0 %: 2,2',3,3'-Tetrafluor-4-butoxy-4'-propyltolan
- 4,0 %: 2,2',3,3'-Tetrafluor-4-butoxy-4'-pentyltolan
- 13,0 %: 2,3-Difluor-4-ethoxy-4'-propyltolan
- 13,0 %: 2,3-Difluor-4-ethoxy-4'-pentyltolan
- 5,0 %: 4-(trans-4-Propylcyclohexyl)-4'-methoxytolan
- 5,0 %: 4-(trans-4-Propylcyclohexyl)-4'-ethoxytolan
- 5,0 %: 4-(trans-4-Propylcyclohexyl)-4'-propoxytolan
- 3,0 %: trans,trans-4-Propylcyclohexylcyclohexan-4'-ylcarbonsäure-(2,3-difluor-4-ethoxyphenylester)
- 5,0 %: 4-(trans,trans-4-Propylcyclohexylcyclohexan-4'-yl)-2,3-difluor-ethoxybenzol
- 4,0 %: 2-(p-Pentylphenyl)-6-fluor-5-ethoxypyridin

Die physikalischer Eigenschaften der Medien für ECB-Anzeigen der Beispiele D, E und F können der folgenden Tabelle I entnommen werden:

**Tabelle I**

| Beispiel | D | E | F |
|---|---|---|---|
| Klärpunkt (°C) | +83 | +84 | +83 |
| Viskosität (mm²s⁻¹) bei 20°C | 25 | < 25 | < 25 |
| Δn | +0,218 | +0,217 | +0,214 |
| V_{(90,0,20)} (V) | 2,4 | 2,8 | 2,8 |
| V_{(10,0,20)} (V) | 2,6 | 2,9 | 2,9 |

## Patentansprüche

1. 3,6-Disubstituierte-2-Fluoropyridine der Formel I, 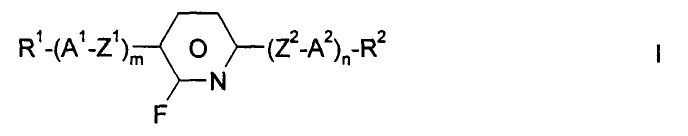 wobei
R¹ und R² jeweils unabhängig voneinander einen unsubstituierten oder einen einfach durch CN oder Halogen substituierten Alkyl- oder Alkenylrest mit bis zu 15 C-Atomen, wobei in diesen Resten auch eine oder mehrere CH₂-Gruppen jeweils unabhängig voneinander durch -S-, -O-, -CO-, - CO-O-, -O-CO- oder -O-CO-O- so ersetzt sein können, daß S- und/oder O-Atome nicht direkt miteinander verknüpft sind, einer der Reste R¹ und R² auch Halogen, - CF₃, -OCF₃ oder OCHF₂,
A¹ und A² jeweils unabhängig voneinander einen
(a) trans-1,4-Cyclohexylenrest, worin auch eine oder mehrere nicht benachbarte CH₂-Gruppen durch -O- und/oder -S-ersetzt sein können,
(b) 1,4-Phenylenrest, worin auch eine oder zwei CH-Gruppen durch N ersetzt sein können,
wobei der Rest (a) durch CN oder Halogen und der Rest (b) durch Halogen substituiert sein können,
Z¹ und Z² jeweils unabhängig voneinander -CH₂-CH₂-, -C≡C-, -CH₂O-, -OCH₂-, -CO-O-, -O-CO-, -CH=N-, -CH₂S-, -SCH₂- ,eine Einfachbindung oder eine Alkylengruppe mit 3 bis 6 C-Atomen, worin auch eine CH₂-Gruppe durch -O-, -CO-O-, -O-CO-, -CH-Halogen- oder -CHCN-ersetzt sein kann,
m 0, 1 oder 2,
n 0, 1 oder 2, und
m + n 1, 2 oder 3
bedeuten,
mit den Maßgaben, daß
a) im Falle R² Halogen, -CF₃, -OCF₃, OCHF₂ oder Alkoxy,
n 1 oder 2 ist,
b) im Falle n = 1 oder 2,
Z² -CH₂CH₂-, -C≡C-, -CH₂O-, -CO-O- oder eine Einfachbindung bedeutet, und
c) Verbindungen ausgeschlossen sind, in denen R² Alkenyloxy, Oxaalkyloxy oder Alkylcarbonyloxy ist und n 0 bedeutet.

2. Fluoropyridine nach Anspruch 1,
worin
n 1 oder 2 ist, und mindestens einer der Reste A¹ und A² gegebenenfalls durch Fluor substituiertes 1,4-Phenylen, 1,4-Cyclohexylen, Pyrimidin-2,5-diyl oder Pyridin-2,5-diyl bedeutet.

3. Verwendung der Verbindungen der Formel I nach Anspruch 1 als Komponenten flüssigkristalliner Medien für elektrooptische Anzeigeelemente.

4. Flüssigkristallines Medium mit mindestens zwei Komponenten, dadurch gekennzeichnet, daß mindestens eine Komponente eine Verbindung der Formel I nach Anspruch 1 ist.

5. Ferroelektrisches flüssigkristallines Medium mit mindestens einer chiralen Komponente und mindestens einer achiralen Komponente, dadurch gekennzeichnet, daß mindestens eine Komponente eine flüssigkristalline Verbindung der Formel I nach Anspruch 1 enthält.

6. Elektrooptische Anzeige, dadurch gekennzeichnet, daß sie als Dielektrikum ein flüssigkristallines Medium nach Anspruch 4 enthält.

7. Matrix-Flüssigkristallanzeige, dadurch gekennzeichnet, daß sie als Dielektrikum ein flüssigkristallines Medium nach Anspruch 4 enthält.

8. Flüssigkristall-Schalt- und Anzeigevorrichtung, enthaltend ein ferroelektrisches flüssigkristallines Medium, Trägerplatten, Elektroden, mindestens eine Orientierungsschicht sowie gegebenenfalls zusätzliche Hilfsschichten, dadurch gekennzeichnet, daß das Medium mindestens eine flüssigkristalline Verbindung Formel I nach Anspruch 1 enthält.

9. Flüssigkristallines Medium nach Anspruch 4, dadurch gekennzeichnet, daß es nematisch ist.

10. ECB-Anzeige, dadurch gekennzeichnet, daß sie ein flüssigkristallines Medium nach Anspruch 9 enthält.

## Claims

1. 3,6-Disubstituted 2-fluoropyridines of the formula I 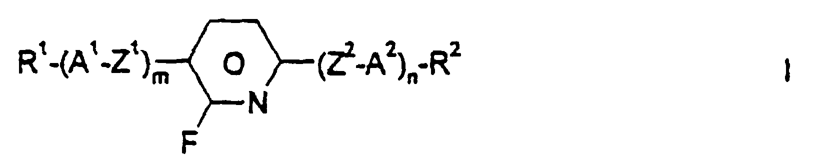 where
R¹ and R² are each, independently of one another, an alkyl or alkenyl radical having up to 15 carbon atoms which is unsubstituted or monosubstituted by CN or halogen, it also being possible for one or more CH₂ groups in these radicals to be replaced, in each case independently of one another, by -S-, -O-, -CO-, -CO-O-, -O-CO- or -O-CO-O- in such a manner that S and/or O atoms are not linked directly to one another, and one of the radicals R¹ and R² is alternatively halogen, -CF₃, -OCF₃ or OCHF₂,
A¹ and A² are each, independently of one another, a
(a) trans-1,4-cyclohexylene radical in which, in addition, one or more non-adjacent CH₂ groups may be replaced by -O- and/or -S-,
(b) 1,4-phenylene radical in which, in addition, one or two CH groups may be replaced by N,
it being possible for the radical (a) to be substituted by CN or halogen and the radical (b) to be substituted by halogen,
Z¹ and Z² are each, independently of one another, -CH₂-CH₂-, -C≡C-, -CH₂O-, -OCH₂-, -CO-O-, -O-CO-, -CH=N-, -CH₂S-, -SCH₂-, a single bond or an alkylene group having 3 to 6 carbon atoms in which, in addition, one CH₂ group may be replaced by -O-, -CO-O-, -O-CO-, -CH-halogen- or -CHCN-,
m is 0, 1 or 2,
n is 0, 1 or 2, and
m + n is 1, 2 or 3,
with the provisos that
a) in the case where R² is halogen, -CF₃, -OCF₃, OCHF₂ or alkoxy,
n is 1 or 2,
b) in the case where n = 1 or 2,
Z² is -CH₂CH₂-, -C≡C-, -CH₂O-, -CO-O- or a single bond
c) compounds are excepted where R² is alkenyloxy, oxaalkyloxy or alkylcarbonyloxy and n is O.

2. Fluoropyridines according to Claim 1, in which
n is 1 or 2, and at least one of the radicals A¹ and A² is optionally fluorine-substituted 1,4-phenylene, 1,4-cyclohexylene, pyrimidine-2,5-diyl or pyridine-2,5-diyl.

3. Use of the compounds of the formula I according to Claim 1 as components of liquid-crystalline media for electrooptical display elements.

4. Liquid-crystalline medium having at least two components, characterized in that at least one component is a compound of the formula I according to Claim 1.

5. Ferroelectric liquid-crystalline medium having at least one chiral component and at least one achiral component, characterized in that at least one component contains a liquid-crystalline compound of the formula I according to Claim 1.

6. Electrooptical display, characterized in that it contains, as dielectric, a liquid-crystalline medium according to Claim 4.

7. Matrix liquid-crystal display, characterized in that it contains, as dielectric, a liquid-crystalline medium according to Claim 4.

8. Liquid-crystal switching and display device, containing a ferroelectric liquid-crystalline medium, outer plates, electrodes, at least one alignment layer and optionally additional assistant layers, characterized in that the medium contains at least one liquid-crystalline compound of the formula I according to Claim 1.

9. Liquid-crystalline medium according to Claim 4, characterized in that it is nematic.

10. ECB display, characterized in that it contains a liquid-crystalline medium according to Claim 9.

## Revendications

1. 2-fluoropyridines 3,6-disubstituées de formule I 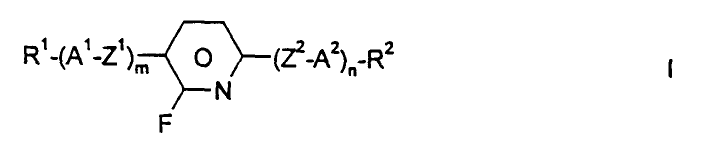 dans laquelle
R¹ et R² représentent chacun indépendamment l'un de l'autre un radical alkyle ou alcényle ayant jusqu'à 15 atomes de carbone, non substitué ou monosubstitué par CN ou un halogène, où dans ces radicaux un ou plusieurs groupes CH₂ peuvent à chaque fois être remplacés de manière indépendante par -S-, -O-, -CO-, -CO-O-, -O-CO-ou -O-CO-O- de manière que les atomes de S et/ou de O ne soient pas directement reliés entre eux, l'un des radicaux R¹ et R² pouvant également représenter un halogène, -CF₃, -OCF₃ ou OCHF₂ ,
A¹ et A² représentent chacun indépendamment l'un de l'autre
(a) un radical trans-1,4-cyclohexylène, où également un ou plusieurs groupes CH₂ non voisins peuvent être représentés par -O- et/ou -S-,
(b) un radical 1,4-phénylène, où également un ou deux groupes CH peuvent être remplacés par N, le radical (a) pouvant être substitué par CN ou un halogène et le radical (b) par un halogène,
Z¹ et Z² représentent chacun indépendamment l'un de l'autre -CH₂-CH₂-, -C≡C-, -CH₂O-, -OCH₂-, -CO-O-, -O-CO-, -CH=N-, -CH₂S-, -SCH₂-, une liaison simple ou un groupe alkylène ayant de 3 à 6 atomes de carbone, où également un groupe CH₂ peut être remplacé par -O-, -CO-O-, -O-CO-, -CH-halogène ou -CHCN-,
m vaut 0, 1 ou 2,
n vaut 0, 1 ou 2, et
m + n vaut 1, 2 ou 3,
sous réserve que
a) dans le cas où R² est un halogène, -CF₃, -OCF₃, OCHF₂ ou un alcoxy,
n vaut 1 ou 2,
b) dans le cas où n = 1 ou 2,
Z² représente -CH₂CH₂-, -C≡C-, -CH₂O-, -CC-O- ou une liaison simple, et
c) sont exclus les composés dans lesquels R² représente un alcényloxy, un oxaalkyloxy ou un alkylcarbonyloxy et n vaut 0.

2. Fluoropyridines selon la revendication 1,
dans lesquelles
n vaut 1 ou 2, et au moins un des radicaux A¹ et A² représente un 1,4-phénylène, 1,4-cyclohexylène, pyrimidine-2,5-diyle ou pyridine-2,5-diyle éventuellement substitué par un fluor.

3. Utilisation des composés de formule I selon la revendication 1 comme composants de milieux mésomorphes (à cristaux liquides) pour éléments d'affichage électro-optiques.

4. Milieu mésomorphe comportant au moins deux composants, caractérisé en ce qu'au moins un composant est un composé de formule I selon la revendication 1.

5. Milieu mésomorphe ferroélectrique avec au moins un composant chiral et au moins un composant achiral, caractérisé en ce qu'au moins un composant contient un composé mésomorphe de formule I selon la revendication 1.

6. Affichage électro-optique caractérisé en ce qu'il contient comme diélectrique un milieu mésomorphe selon la revendication 4.

7. Affichage mésomorphe à matrice, caractérisé en ce qu'il contient comme diélectrique un milieu mésomorphe selon la revendication 4.

8. Dispositif de commutation et d'affichage mésomorphe contenant un milieu mésomorphe ferro-électrique, des plaques support, des électrodes, au moins une couche d'orientation, ainsi que le cas échéant des couches auxiliaires additionnelles, caractérisé en ce que le milieu contient au moins un composé mésomorphe de formule I selon la revendication 1.

9. Milieu mésomorphe selon la revendication 4, caractérisé en ce qu'il est nématique.

10. Affichage ECB caractérisé en ce qu'il contient un milieu mésomorphe selon la revendication 9.
